# EUROPEAN PATENT APPLICATION

(11) **EP 3 206 145 A1**
(43) Date of publication of application: **16.08.2017**
(21) Application number: 16154837.5
(22) Date of filing: 09.02.2016
(51) Int. Cl.: G06F 19/00

(54) **METHOD FOR PRODUCING A TOPICAL DERMAL FORMULATION FOR COSMETIC USE**

(71) Applicant: InnovativeHealth Group SL, 28028 Madrid (ES)
(72) Inventor: García Medel, Noel, 28028 Madrid (ES); Muñoz Molina, Eduardo, 28003 Madrid (ES); Millán Ortega, Estrella, 28049 Madrid (ES)
(74) Representative: Illescas, Manuel

(57) **Abstract**

The present invention relates to a method for producing at least one topical dermal formulation for cosmetic use, comprising one of two alternative algorithms to create novel formulations:
(i) a light method based on a stochastic search system that starts from a random selection of ingredients and seeks to improve the activity of the corresponding formulation by replacing, adding and removing ingredients; or
(ii) a genetic algorithm that starts from a random population of formulations which are optimized by iteration according to their intended cosmetic use.

## Description

### Field of the Invention

The present invention relates to a method for producing cosmetics, in particular to a method for producing a topical dermal formulation for cosmetic use.

### Background to the Invention

Analysis of the utility of cosmetic ingredients for any given application is usually oriented to their previously known applicability, their biological activity and other properties such as skin permeability.

However, no precedents for tools providing automated rational design of dermocosmetics exist, let alone tools which include a validated description of the biological activity of each of the ingredients, particularly when said ingredients comprise complex mixtures of compounds. In contrast, the only tools currently available are merely aimed at assisting in the design of novel cosmetic products. As one example, Coptis provides the Coptis Lab tool which uses a cosmetic ingredients database that includes regulatory affairs corresponding to the legal frames of the major markets for the cosmetics industry. Another example is the tool provided by BASF for the calculation of the sun protection factor (SPF) for formulations proposed by the user using ingredients supplied by BASF. The report that is generated therefrom fits specific regulatory affairs for the requested market. Thus, the aforementioned tools have a very limited scope and do not provide automated rational design of topical dermal formulations for cosmetic use.

It is therefore a problem of the present invention to provide a method for the rational design of topical dermal formulations for cosmetic use. For any given formulation, said method preferably provides a whole activity profile against all targets within a given platform, rather than necessitating an application-specific confirmatory assay, and provides maximum biological activity against the intended biological targets, thereby providing optimal efficacy for a given cosmetic application. Thus, it is a further problem of the present invention to provide a method that improves the efficiency of the formulation development. The present invention is intended to meet these demands.

### Brief Description of the Invention

The present invention relates to a method for producing at least one topical dermal formulation for a given cosmetic use, comprising the following steps:
**(a)** determining the IC₅₀ of ingredients which exhibit antagonistic biological activity against at least one target which, when inhibited, effects a cosmetic use, and/or determining the EC₅₀ of ingredients which exhibit agonistic biological activity against at least one target which, when activated, effects a cosmetic use, wherein each of said ingredients may be a compound or a composition, and wherein said target is selected from at least one molecule, protein, protein complex, nucleic acid or nucleic acid complex;
**(b)** compiling an ingredient database comprising data on each of said ingredients, wherein the data for any given ingredient encodes at least one qualitative characteristic of said ingredient and at least one quantitative characteristic of said ingredient, wherein:
   - a qualitative characteristic is selected from a qualitative chemical property of said given ingredient, a qualitative physical property of said given ingredient, an organoleptic property of said given ingredient, a supplier of said given ingredient, or a dermatological test performed using said given ingredient; and
   - a quantitative characteristic is selected from an IC₅₀ or EC₅₀ value of said given ingredient determined in step (a), a skin permeability value of said given ingredient, a cost per unit weight of said given ingredient, a quantitative chemical property of said given ingredient, or a quantitative physical property of said given ingredient;
**(c)** either:
   **(c1)** identifying those ingredients which have data encoding at least one first given qualitative characteristic of said ingredients in said ingredient database, wherein said first given qualitative characteristic is selected from a presence or absence of a given qualitative chemical property, a presence or absence of a given qualitative physical property, a presence or absence of a given organoleptic property, the provenance or not from a given supplier, or a presence or absence of passing a given dermatological test performed;
   **(c2)**
      **(c2a)** making a random selection of two or more of said ingredients identified in step (c1) for combination into a formulation for said given cosmetic use; and
      **(c2b)** calculating the total cost per unit weight of said formulation when all ingredients in said random selection of ingredients made in step (c2a) exhibit:
         - antagonistic biological activity (AN) against a given target or targets, wherein each given target is that which, when inhibited in an experimental skin model, effects said given cosmetic use, or
         - agonistic biological activity (AG) against a given target or targets, wherein each given target is that which, when activated in an experimental skin model, effects said given cosmetic use,
            by multiplying the cost per unit weight of each ingredient in the random selection made in step (c2a) by the concentration (C) at which it is to be combined into said formulation, wherein the concentration (C) for any given ingredient in said random selection is:
         - the IC₅₀ value of said ingredient determined in step (a) against said given target, or the maximum of the IC₅₀ values of said ingredient determined in step (a) against said given targets, when said ingredient exhibits said antagonistic biological activity (AN), or
         - the EC₅₀ value of said ingredient determined in step (a) against said given target, or the maximum of the EC₅₀ values of said ingredient determined in step (a) against said given targets, when said ingredient exhibits said agonistic biological activity (AG); and
      **(c2c)** randomly varying said random selection if the formulation to be made from combining said random selection of ingredients at the concentrations (C) referred to in step (c2b):
         - comprises an ingredient that does not have at least one second given qualitative characteristic, wherein said second given qualitative characteristic is different from the at least one first given qualitative characteristic in step (c1), and wherein said second given qualitative characteristic is selected from a presence or absence of a given qualitative chemical property, a presence or absence of a given qualitative physical property, a presence or absence of a given organoleptic property, a presence or absence of a given supplier, or a presence or absence of passing a given dermatological test performed;
         - comprises an ingredient which does not exhibit the antagonistic biological activity (AN) or the agonistic biological activity (AG) referred to in step (c2b); and/or
         - does not have a total cost per unit weight calculated according to step (c2b) which is less than a given total cost per unit weight,
            by:
            **(c2ci)** randomly deselecting one of said ingredients from said random selection, provided that said random selection comprises more than two ingredients, thus decreasing the number of ingredients for combination into said formulation;
            **(c2cii)** randomly selecting a further ingredient identified in step (c1) for combination with said random selection, thus increasing the number of ingredients for combination into said formulation;
            **(c2ciii)** randomly deselecting one of said ingredients from said random selection, and randomly selecting another ingredient identified in step (c1), for combination with the remaining ingredient or ingredients of said random selection, thus maintaining constant the number of ingredients for combination into said formulation; or
            **(c2civ)** randomly deselecting a number, x, of said ingredients from said random selection, and randomly selecting a further x ingredients from any of the random selections generated in step (c5), for combination with the remaining ingredient or ingredients of said random selection, provided that said random selection comprises more than (x + 1) ingredients, thus maintaining constant the number of ingredients for combination into said formulation; and
      **(c2d)** repeating steps (c2b) and (c2c) n times, wherein n is a whole number less than 100, until:
         - all ingredients comprised in said random selection of ingredients have said at least one second given qualitative characteristic,
         - all ingredients comprised in said random selection of ingredients exhibit the antagonistic biological activity (AN) or the agonistic biological activity (AG) referred to in step (c2b), and
         - said formulation to be made from combining said random selection of ingredients has a total cost per unit weight calculated according to step (c2b) which is less than said given total cost per unit weight;
   **(c3)** determining the score of said random selection of two or more ingredients for combination into said formulation, wherein said score is determined by:
      - averaging the mean score of each of the ingredients comprised in said random selection, wherein said mean score of any given ingredient is determined by averaging the percentage of a value of at least one quantitative characteristic of said given ingredient at the concentration (C) referred to in step (c2b), relative to a corresponding predetermined value of said at least one quantitative characteristic, and/or determining the percentage of a value of at least one quantitative characteristic of said formulation relative to a corresponding predetermined value of said at least one quantitative characteristic; and/or
      - averaging the mean score of each of the ingredients comprised in said random selection, wherein said mean score of any given ingredient is determined by averaging the percentage of an inverse value of at least one quantitative characteristic of said given ingredient at the concentrations (C) referred to in step (c2b), relative to an inverse of a corresponding predetermined value of said at least one quantitative characteristic, and/or determining the percentage of an inverse value of at least one quantitative characteristic of said formulation relative to an inverse of a corresponding predetermined value of said at least one quantitative characteristic;
      wherein said quantitative characteristic of each ingredient is selected from an IC₅₀ or EC₅₀ value of said ingredient determined in step (a), a skin permeability value of said ingredient, a cost per unit weight of said ingredient, a quantitative chemical property of said ingredient, or a quantitative physical property of said ingredient, and
      wherein said quantitative characteristic of said formulation is selected from a total cost per unit weight of said formulation, a total number of ingredients comprised in said formulation, an IC₅₀ or EC₅₀ value of said formulation against at least one target, a quantitative chemical property of said formulation, or a quantitative physical property of said formulation, and
      wherein when an ingredient in said random selection of two or more ingredients does not exhibit a given quantitative characteristic, the percentage assigned thereto is zero;
   **(c4)** compiling a formulation database comprising data on said random selection made in step (c2), wherein said data encodes:
      - the type and number of ingredients comprised in said random selection and their respective quantities;
      - the score of said random selection determined in step (c3);
      - the total cost per unit weight of said formulation to be made from combining said random selection of ingredients; and
      - the qualitative characteristics and quantitative characteristics of the ingredients comprised therein, wherein said qualitative characteristics and said quantitative characteristics are those compiled in step (b);
   **(c5)**
      **(c5a)** randomly varying said random selection for which data is compiled in said formulation database to generate a new random selection for combination into a new formulation for said given cosmetic use by:
         **(c5ai)** randomly deselecting one of said ingredients from said random selection, provided that said given random selection comprises more than two ingredients, thus decreasing the number of ingredients for combination into said formulation;
         **(c5aii)** randomly selecting a further ingredient identified in step (c1) for combination with said random selection, thus increasing the number of ingredients for combination into said formulation;
         **(c5aiii)** randomly deselecting one of said ingredients from said random selection, and randomly selecting a further ingredient identified in step (c1), for combination with the remaining ingredient or ingredients of said random selection, thus maintaining constant the number of ingredients for combination into said formulation; or
         **(c5aiv)** randomly deselecting a number, x, of said ingredients from said random selection, and randomly selecting a further x ingredients from any of the random selections generated in step (c8), for combination with the remaining ingredient or ingredients of said random selection, provided that said random selection comprises more than (x + 1) ingredients, thus maintaining constant the number of ingredients for combination into said formulation;
      **(c5b)** calculating the total cost per unit weight of said new formulation according to step (c2b), provided that all ingredients comprised in said new random selection of two or more ingredients obtained in step (c5a) exhibit the antagonistic biological activity (AN) or the agonistic biological activity (AG) referred to in step (c2b); and
      **(c5c)** determining the score of said new random selection of two or more ingredients obtained in step (c5a) for combination into said new formulation according to step (c3);
   **(c6)** replacing the data on said random selection in said formulation database with the corresponding data on said new random selection if:
      - the score of said new random selection determined in step (c5c) is greater than the score of said random selection in said formulation database;
      - said new random selection comprises an ingredient that has the at least one second given qualitative characteristic referred to in step (c2c);
      - said new random selection comprises an ingredient which exhibits the antagonistic biological activity (AN) or the agonistic biological activity (AG) referred to in step (c2b); and/or
      - said new random selection has a total cost per unit weight calculated according to step (c5b) which is less than said given total cost per unit weight,
   **(c7)** repeating steps (c5) and (c6) N times, wherein N is a whole number less than 100;
   **(c8)** optionally repeating steps (c2) to (c7) S times to afford S random selections of two or more of said ingredients for combination into S respective formulations, wherein S is a whole number less than 1000;
      or
   **(c9)** identifying those ingredients which have data encoding at least one given qualitative characteristic of said ingredients in said ingredient database wherein, for a given ingredient, said given qualitative characteristic is selected from a presence or absence of a given qualitative chemical property of said given ingredient, a presence or absence of a given qualitative physical property of said given ingredient, a presence or absence of a given organoleptic property of said given ingredient, or the provenance or not from a given supplier of said given ingredient;
   **(c10)** generating 2^{T} binary sequences, wherein each binary sequence reads from left to right and comprises a tuple, T, of bits, wherein T is calculated by rounding up to the nearest whole number the result of log₂(NI), wherein NI is the number of ingredients identified in step (c9), wherein each bit is additionally assigned a value between 0 and 0.1 corresponding to the probability that said bit changes;
   **(c11)** assigning each of the ingredients identified in step (c9) one of the binary sequences generated in step (c10);
   **(c12)** generating a population of S composite binary sequences, wherein each composite binary sequence reads from left to right and comprises P binary sequences reading from left to right and joined in linear sequence, and wherein
      - each of the P binary sequences is randomly selected from the 2^{T} binary sequences generated in step (c10);
      - S is a whole number less than 1000; and
      - P is a given whole number between 2 and 20 which refers to the maximum possible number of ingredients in said formulation for said given cosmetic use;
   **(c13)** for each composite binary sequence:
      **(c13a)** identifying which of the ingredients identified in step (c9) are encoded by the P binary sequences comprised therein for combination into a formulation encoded by said composite binary sequence;
      **(c13b)** calculating the total cost per unit weight of the formulation encoded by said composite binary sequence, provided that at least two ingredients were identified in step (c13a) and all ingredients identified in step (c13a) exhibit:
         - antagonistic biological activity (AN) against a given target or targets, wherein each given target is that which, when inhibited in an experimental skin model, effects said given cosmetic use, or
         - agonistic biological activity (AG) against a given target or targets, wherein each given target is that which, when activated in an experimental skin model, effects said given cosmetic use,
         by multiplying the cost per unit weight of each ingredient identified in step (c13a) by the concentration (C) at which it is to be combined into the formulation encoded by said composite binary sequence, wherein the concentration (C) for any given ingredient identified in step (c13a) is:
         - the IC₅₀ value of said given ingredient as determined in step (a) against said given target, or the maximum of the IC₅₀ values of said ingredient determined in step (a) against said given targets, when said ingredient exhibits said antagonistic biological activity (AN), or
         - the EC₅₀ value of said given ingredient as determined in step (a) against said given target, or the maximum of the EC₅₀ values of said ingredient determined in step (a) against said given targets, when said ingredient exhibits said agonistic biological activity (AG); and
      **(c13c)** determining the score of the formulation encoded by said composite binary sequence, wherein said score is determined by:
         - averaging the mean score of each of the ingredients identified in step (c13a), wherein said mean score of any given ingredient is determined by averaging the percentage of a value of at least one quantitative characteristic of said given ingredient relative to a corresponding predetermined value of said at least one quantitative characteristic, and/or determining the percentage of a value of at least one quantitative characteristic of the formulation encoded by said composite binary sequence relative to a corresponding predetermined value of said at least one quantitative characteristic; and/or
         - averaging the mean score of each of the ingredients identified in step (c13a), wherein said mean score of any given ingredient is determined by averaging the percentage of an inverse value of at least one quantitative characteristic of said given ingredient relative to an inverse of a corresponding predetermined value of said at least one quantitative characteristic, and/or determining the percentage of an inverse value of at least one quantitative characteristic of the formulation encoded by said composite binary sequence relative to an inverse of a corresponding predetermined value of said at least one quantitative characteristic;
         wherein said quantitative characteristic of each ingredient identified in step (c13a) is selected from an IC₅₀ or EC₅₀ value of said ingredient determined in step (a), a skin permeability value of said ingredient, a cost per unit weight of said ingredient, a quantitative chemical property of said ingredient or a quantitative physical property of said ingredient, and
         wherein said quantitative characteristic of the formulation encoded by said composite binary sequence is selected from a cost per unit weight of said formulation, a total number of ingredients comprised in said formulation, an IC₅₀ or EC₅₀ value of said formulation against at least one target, a quantitative chemical property of said formulation, or a quantitative physical property of said formulation and
         wherein when:
         - an ingredient identified in step (c13a) does not exhibit a given quantitative characteristic, the percentage assigned thereto is zero; and
         - less than two ingredients were identified in step (c13a) the score of the formulation encoded by said composite binary sequence is zero;
   **(c14)** compiling a composite binary sequences database comprising data on each of the composite binary sequences in said population of S composite binary sequences, wherein, for any given composite binary sequence, said data encodes
      - the type and number of ingredients in the formulation encoded by said composite binary sequence and their respective quantities;
      - the score of said binary sequence determined in step (c13c);
      - the cost per unit weight of the formulation encoded by said composite binary sequence; and
      - the qualitative characteristics and quantitative characteristics of the ingredients in the formulation encoded by said composite binary sequence, wherein said qualitative characteristics and said quantitative characteristics are those compiled in step (b);
   **(c15)**
      **(c15a)** duplicating the sub-population of composite binary sequences which encode a formulation having a score in the upper 50% of the population of S formulations;
      **(c15b)** randomly interchanging the terminal F% of bits in each composite binary sequence that is comprised in said duplicate sub-population of composite binary sequences with the terminal F% of bits from any other composite binary sequence in said duplicate sub-population of composite binary sequences, thus generating S/2 new composite binary sequences;
      **(c15c)** replacing the data for the sub-population of composite binary sequences in said composite binary sequence database which encode a formulation having a score in the lower 50% of the population of S formulations, with data on S/2 new composite binary sequences which were generated in step (c15b);
      **(c15d)** optionally varying the value of one randomly selected bit in each composite binary sequence comprised in
         - the sub-population of composite binary sequences which encode a formulation having a score in the upper 50% of the population of S formulations, and
         - the S/2 new composite binary sequences generated in step (c15b), wherein the probability that any given bit changes value is between 0 and 0.1, thus generating a new population of S composite binary sequences;
   **(c16)** repeating steps (c13) and (c14) for the new population of S composite binary sequences generated in step (c15);
   **(c17)** repeating steps (c15) and (c16) K times, wherein K is a whole number less than 1000, or until the average score of the formulations encoded by said composite binary sequence is not increased between consecutive repetitions of steps (c15) and (c16);
**(d)**
   **(d1)** selecting:
      - the random selection for combination into a formulation obtained in step (c7); or
      - the M percent of the random selections for combination into formulations obtained in step (c8) having the highest scores, wherein M is a whole number from 1 to 50,
      as the random selection or random selections of ingredients for combination into said at least one topical dermal formulation for cosmetic use, or
   **(d2)** selecting:
      - the composite binary sequence obtained in step (c17) encoding the formulation having the highest score; or
      - the M percent of the composite binary sequences obtained in step (c17) encoding the formulations having the highest scores, wherein M is a whole number from 1 to 50,
      as the composite binary sequence or composite binary sequences encoding said at least one topical dermal formulation for cosmetic use; and
**(e)** making said at least one topical dermal formulation for cosmetic use by mixing the ingredients:
   - comprised in the random selection or random selections, or
   - encoded by the composite binary sequence or composite binary sequences selected in step (d).

The present invention preferably also relates to a method for producing at least one topical dermal formulation for a given cosmetic use selected from an anti-cellulitis use, an anti-aging use, an anti-rhytid use, an atopic skin care use, a dark circles attenuation use, a hair growth stimulation use, a moisturizing use, a tissue refirming use, a sensitive skin care use, a skin repair use, a skin whitening or sun block/sun care use, comprising the following steps:
**(a)** determining the IC₅₀ of ingredients which exhibit antagonistic biological activity against at least one target in an experimental *in vitro* skin cell model which, when inhibited, effects a cosmetic use, and/or determining the EC₅₀ of ingredients which exhibit agonistic biological activity against at least one target in an experimental *in vitro* skin cell model which, when activated, effects a cosmetic use, wherein each of said ingredients may be a compound or a composition, wherein said target is selected from at least one organic compound, protein or protein complex, and wherein said biological activity against a target is selected from NF-κB activity in keratinocytes, NF-κB activity in fibroblasts, PPARγ activity, PPARα activity, induction of Nrf2 activity, inhibition of Nrf2 activity, total antioxidant activity, cellular antioxidant activity, TRPV-1 agonistic activity or TRPV-1 antagonistic activity;
**(b)** compiling an ingredient database comprising data on each of said ingredients, wherein the data for any given ingredient encodes at least one qualitative characteristic of said ingredient and at least one quantitative characteristic of said ingredient, wherein:
   - a qualitative characteristic is selected from a qualitative physicochemical property of said given ingredient, an organoleptic property of said given ingredient, a supplier of said given ingredient, or a dermatological test performed using said given ingredient; and
   - a quantitative characteristic is selected from an IC₅₀ or EC₅₀ value of said given ingredient determined in step (a), a skin permeability value of said given ingredient, a cost per unit weight of said given ingredient, or a quantitative physicochemical property of said given ingredient;
**(c)** either:
   **(c1)** identifying those ingredients which have data encoding at least one first given qualitative characteristic of said ingredients in said ingredient database, wherein said first given qualitative characteristic is selected from a presence or absence of a given qualitative physicochemical property, a presence or absence of a given organoleptic property, the provenance or not from a given supplier, or a presence or absence of passing a given dermatological test performed;
   **(c2)**
      **(c2a)** making a random selection of between 2 and 20 of said ingredients identified in step (c1) for combination into a formulation for said given cosmetic use; and
      **(c2b)** calculating the total cost per unit weight of said formulation when all ingredients in said random selection of ingredients made in step (c2a) exhibit:
         - antagonistic biological activity (AN) against a given target or targets, wherein each given target is that which, when inhibited in an experimental *in vitro* skin cell model, effects said given cosmetic use, or
         - agonistic biological activity (AG) against a given target or targets, wherein each given target is that which, when activated in an experimental *in vitro* skin cell model, effects said given cosmetic use,
         by multiplying the cost per unit weight of each ingredient in the random selection made in step (c2a) by the concentration (C) at which it is to be combined into said formulation, wherein the concentration (C) for any given ingredient in said random selection is:
         - the IC₅₀ value of said ingredient determined in step (a) against said given target, or the maximum of the IC₅₀ values of said ingredient determined in step (a) against said given targets, when said ingredient exhibits said antagonistic biological activity (AN), or
         - the EC₅₀ value of said ingredient determined in step (a) against said given target, or the maximum of the EC₅₀ values of said ingredient determined in step (a) against said given targets, when said ingredient exhibits said agonistic biological activity (AG); and
      **(c2c)** randomly varying said random selection if the formulation to be made from combining said random selection of ingredients at the concentrations (C) referred to in step (c2b):
         - comprises an ingredient that does not have at least one second given qualitative characteristic, wherein said second given qualitative characteristic is different from the at least one first given qualitative characteristic in step (c1), and wherein said second given qualitative characteristic is selected from a presence or absence of a given qualitative physicochemical property, a presence or absence of a given organoleptic property, a presence or absence of a given supplier, or a presence or absence of passing a given dermatological test performed;
         - comprises an ingredient which does not exhibit the antagonistic biological activity (AN) or the agonistic biological activity (AG) referred to in step (c2b); and/or
         - does not have a total cost per unit weight calculated according to step (c2b) which is less than a given total cost per unit weight,
            by:
            **(c2ci)** randomly deselecting one of said ingredients from said random selection, provided that said random selection comprises more than two ingredients, thus decreasing the number of ingredients for combination into said formulation;
            **(c2cii)** randomly selecting a further ingredient identified in step (c1) for combination with said random selection, thus increasing the number of ingredients for combination into said formulation;
            **(c2ciii)** randomly deselecting one of said ingredients from said random selection, and randomly selecting another ingredient identified in step (c1), for combination with the remaining ingredient or ingredients of said random selection, thus maintaining constant the number of ingredients for combination into said formulation; or
            **(c2civ)** randomly deselecting a number, x, of said ingredients from said random selection, and randomly selecting a further x ingredients from any of the random selections generated in step (c5), for combination with the remaining ingredient or ingredients of said random selection, provided that said random selection comprises more than (x + 1) ingredients, thus maintaining constant the number of ingredients for combination into said formulation; and
      **(c2d)** repeating steps (c2b) and (c2c) n times, wherein n is a whole number less than 100, until:
         - all ingredients comprised in said random selection of ingredients have said at least one second given qualitative characteristic,
         - all ingredients comprised in said random selection of ingredients exhibit the antagonistic biological activity (AN) or the agonistic biological activity (AG) referred to in step (c2b), and
         - said formulation to be made from combining said random selection of ingredients has a total cost per unit weight calculated according to step (c2b) which is less than said given total cost per unit weight;
   **(c3)** determining the score of said random selection of two or more ingredients for combination into said formulation, wherein said score is determined by:
      **(c3a)** averaging the mean score of each of the ingredients comprised in said random selection, wherein said mean score of any given ingredient is determined by averaging the following parameters:
         (i) the percentage of:
            - an assigned value based on the IC₅₀ value of said given ingredient in said formulation against at least one given target, relative to a maximum possible assigned value, wherein a value of 0 may be assigned to said given ingredient against a given target when it is not inhibited at any concentration *in vitro,* a value of 1 may be assigned to said given ingredient when inhibition is observed at a concentration falling above the upper quartile of the IC₅₀ values for each ingredient in all of said n formulations against said at least one given target; a value of 2 may be assigned to said given ingredient when inhibition is observed at a concentration falling between the upper and lower quartiles of the IC₅₀ values for each ingredient in all of said n formulations against said at least one given target, and a value of 3 (the maximum possible assigned value) may be assigned when inhibition is observed at a concentration falling below the lower quartile of the IC₅₀ values for each ingredient in all of said n formulations against said at least one given target; or
            - the inverse of the IC₅₀ value of said given ingredient in said formulation against at least one given target, relative to the inverse of the maximum IC₅₀ value against said at least one given target for an ingredient in any of said n formulations;
         (ii) the percentage of:
            - an assigned value based on the EC₅₀ value of said given ingredient in said formulation against at least one given target, relative to a maximum possible assigned value, wherein a value of 0 may be assigned to said given ingredient against a given target when it is not activated/induced at any concentration *in vitro,* a value of 1 may be assigned to said given ingredient when activation/induction is observed at a concentration falling above the upper quartile of the EC₅₀ values for each ingredient in all of said n formulations against said at least one given target; a value of 2 may be assigned to said given ingredient when activation/induction is observed at a concentration falling between the upper and lower quartiles of the EC₅₀ values for each ingredient in all of said n formulations against said at least one given target, and a value of 3 (the maximum possible assigned value) may be assigned when activation/induction is observed at a concentration falling below the lower quartile of the EC₅₀ values for an ingredient in any of said n formulations against said at least one given target; or
            - the inverse of the mean EC₅₀ value of said given ingredient in said formulation against at least one given target, relative to the inverse of the maximum EC₅₀ value against said at least one given target for an ingredient in any of said n formulations;
         (iii) the percentage of the inverse cost per unit weight of said ingredient, relative to the inverse cost per unit weight of the highest cost per unit weight ingredient in any of said n formulations; and/or
         (iv) the percentage of the skin permeability value of said ingredient, relative to the maximum skin permeability value for an ingredient in any of said n formulations; and/or
      **(c3b)** calculating:
         (i) the percentage of the number of cosmetic uses of said formulation, relative to the greatest number of cosmetic uses of said n formulations;
         (ii) the percentage of the number of targeted cosmetic uses of said formulation relative to the maximum number of targeted cosmetic uses;
         (iii) the percentage of the number of ingredients identified in step (c1) which are comprised in said formulation, relative to the maximum number of ingredients permissible in said formulation;
         (iv) the percentage of the number of targets against which said formulation exhibits an IC₅₀ or EC₅₀ value, relative to the total number of said targets; and/or
         (v) the percentage of the inverse cost per unit weight of said formulation, relative to the inverse cost per unit weight of the lowest cost per unit weight formulation,
      wherein when an ingredient in said random selection of two or more ingredients does not exhibit a given quantitative characteristic, the percentage assigned thereto is zero;
   **(c4)** compiling a formulation database comprising data on said random selection made in step (c2), wherein said data encodes:
      - the type and number of ingredients comprised in said random selection and their respective quantities;
      - the score of said random selection determined in step (c3);
      - the total cost per unit weight of said formulation to be made from combining said random selection of ingredients; and
      - the qualitative characteristics and quantitative characteristics of the ingredients comprised therein, wherein said qualitative characteristics and said quantitative characteristics are those compiled in step (b);
   **(c5)**
      **(c5a)** randomly varying said random selection for which data is compiled in said formulation database to generate a new random selection for combination into a new formulation for said given cosmetic use by:
         **(c5ai)** randomly deselecting one of said ingredients from said random selection, provided that said given random selection comprises more than two ingredients, thus decreasing the number of ingredients for combination into said formulation;
         **(c5aii)** randomly selecting a further ingredient identified in step (c1) for combination with said random selection, thus increasing the number of ingredients for combination into said formulation;
         **(c5aiii)** randomly deselecting one of said ingredients from said random selection, and randomly selecting a further ingredient identified in step (c1), for combination with the remaining ingredient or ingredients of said random selection, thus maintaining constant the number of ingredients for combination into said formulation; or
         **(c5aiv)** randomly deselecting a number, x, of said ingredients from said random selection, and randomly selecting a further x ingredients from any of the random selections generated in step (c8), for combination with the remaining ingredient or ingredients of said random selection, provided that said random selection comprises more than (x + 1) ingredients, thus maintaining constant the number of ingredients for combination into said formulation;
      **(c5b)** calculating the total cost per unit weight of said new formulation according to step (c2b), provided that all ingredients comprised in said new random selection of two or more ingredients obtained in step (c5a) exhibit the antagonistic biological activity (AN) or the agonistic biological activity (AG) referred to in step (c2b); and **(c5c)** determining the score of said new random selection of two or more ingredients obtained in step (c5a) for combination into said new formulation according to step (c3);
   **(c6)** replacing the data on said random selection in said formulation database with the corresponding data on said new random selection if:
      - the score of said new random selection determined in step (c5c) is greater than the score of said random selection in said formulation database;
      - said new random selection comprises an ingredient that has the at least one second given qualitative characteristic referred to in step (c2c);
      - said new random selection comprises an ingredient which exhibits the antagonistic biological activity (AN) or the agonistic biological activity (AG) referred to in step (c2b); and/or
      - said new random selection has a total cost per unit weight calculated according to step (c5b) which is less than said given total cost per unit weight,
   **(c7)** repeating steps (c5) and (c6) N times, wherein N is a whole number less than 100; and
   **(c8)** optionally repeating steps (c2) to (c7) S times to afford S random selections of two or more of said ingredients for combination into S respective formulations, wherein S is a whole number less than 1000;
      or
   **(c9)** identifying those ingredients which have data encoding at least one given qualitative characteristic of said ingredients in said ingredient database wherein, for a given ingredient, said given qualitative characteristic is selected from a presence or absence of a given qualitative physicochemical property of said given ingredient, a presence or absence of a given organoleptic property of said given ingredient, or the provenance or not from a given supplier of said given ingredient;
   **(c10)** generating 2^{T} binary sequences, wherein each binary sequence reads from left to right and comprises a tuple, T, of bits, wherein T is calculated by rounding up to the nearest whole number the result of log₂(NI), wherein NI is the number of ingredients identified in step (c9), wherein each bit is additionally assigned a value between 0 and 0.1 corresponding to the probability that said bit changes;
   **(c11)** assigning each of the ingredients identified in step (c9) one of the binary sequences generated in step (c10);
   **(c12)** generating a population of S composite binary sequences, wherein each composite binary sequence reads from left to right and comprises P binary sequences reading from left to right and joined in linear sequence, and wherein
      - each of the P binary sequences is randomly selected from the 2^{T} binary sequences generated in step (c10);
      - S is a whole number less than 1000; and
      - P is a given whole number between 2 and 20 which refers to the maximum possible number of ingredients in said formulation for said given cosmetic use;
   **(c13)** for each composite binary sequence:
      **(c13a)** identifying which of the ingredients identified in step (c9) are encoded by the P binary sequences comprised therein for combination into a formulation encoded by said composite binary sequence;
      **(c13b)** calculating the total cost per unit weight of the formulation encoded by said composite binary sequence, provided that at least two ingredients were identified in step (c13a) and all ingredients identified in step (c13a) exhibit:
         - antagonistic biological activity (AN) against a given target or targets, wherein each given target is that which, when inhibited in an experimental *in vitro* skin cell model, effects said given cosmetic use, or
         - agonistic biological activity (AG) against a given target or targets, wherein each given target is that which, when activated in an experimental *in vitro* skin cell model, effects said given cosmetic use,
         by multiplying the cost per unit weight of each ingredient identified in step (c13a) by the concentration (C) at which it is to be combined into the formulation encoded by said composite binary sequence, wherein the concentration (C) for any given ingredient identified in step (c13a) is:
         - the IC₅₀ value of said given ingredient as determined in step (a) against said given target, or the maximum of the IC₅₀ values of said ingredient determined in step (a) against said given targets, when said ingredient exhibits said antagonistic biological activity (AN), or
         - the EC₅₀ value of said given ingredient as determined in step (a) against said given target, or the maximum of the EC₅₀ values of said ingredient determined in step (a) against said given targets, when said ingredient exhibits said agonistic biological activity (AG); and
      **(c13c)** determining the score of the formulation encoded by said composite binary sequence, wherein said score is determined by:
         **(c13ci)** averaging the mean score of each of the ingredients comprised in said formulation identified in step (c13a), wherein said mean score of any given ingredient is determined by averaging the following parameters:
            (i) the percentage of:
               - an assigned value based on the IC₅₀ value of said given ingredient in said formulation against at least one given target, relative to a maximum possible assigned value, wherein a value of 0 may be assigned to said given ingredient against a given target when it is not inhibited at any concentration *in vitro,* a value of 1 may be assigned to said given ingredient when inhibition is observed at a concentration falling above the upper quartile of the IC₅₀ values for each ingredient in all of said n formulations against said at least one given target; a value of 2 may be assigned to said given ingredient when inhibition is observed at a concentration falling between the upper and lower quartiles of the IC₅₀ values for each ingredient in all of said n formulations against said at least one given target, and a value of 3 (the maximum possible assigned value) may be assigned when inhibition is observed at a concentration falling below the lower quartile of the IC₅₀ values for each ingredient in all of said n formulations against said at least one given target; or
               - the inverse of the IC₅₀ value of said given ingredient in said formulation against at least one given target, relative to the inverse of the maximum IC₅₀ value against said at least one given target for an ingredient in any of said n formulations;
            (ii) the percentage of:
               - an assigned value based on the EC₅₀ value of said given ingredient in said formulation against at least one given target, relative to a maximum possible assigned value, wherein a value of 0 may be assigned to said given ingredient against a given target when it is not activated/induced at any concentration *in vitro,* a value of 1 may be assigned to said given ingredient when activation/induction is observed at a concentration falling above the upper quartile of the EC₅₀ values for each ingredient in all of said n formulations against said at least one given target; a value of 2 may be assigned to said given ingredient when activation/induction is observed at a concentration falling between the upper and lower quartiles of the EC₅₀ values for each ingredient in all of said n formulations against said at least one given target, and a value of 3 (the maximum possible assigned value) may be assigned when activation/induction is observed at a concentration falling below the lower quartile of the EC₅₀ values for an ingredient in any of said n formulations against said at least one given target; or
               - the inverse of the mean EC₅₀ value of said given ingredient in said formulation against at least one given target, relative to the inverse of the maximum EC₅₀ value against said at least one given target for an ingredient in any of said n formulations;
            (iii) the percentage of the inverse cost per unit weight of said ingredient, relative to the inverse cost per unit weight of the highest cost per unit weight ingredient in any of said n formulations; and/or
            (iv) the percentage of the skin permeability value of said ingredient, relative to the maximum skin permeability value for an ingredient in any of said n formulations; and/or
      **(c13cii)** calculating:
         (i) the percentage of the number of cosmetic uses of said formulation encoded by said composite binary sequence, relative to the greatest number of cosmetic uses of said n formulations;
         (ii) the percentage of the number of targeted cosmetic uses of said formulation encoded by said composite binary sequence relative to the maximum number of targeted cosmetic uses;
         (iii) the percentage of the number of ingredients identified in step (c9) which are comprised in said formulation encoded by said composite binary sequence, relative to the maximum number of ingredients permissible in said formulation;
         (iv) the percentage of the number of targets against which said formulation encoded by said composite binary sequence exhibits an IC₅₀ or EC₅₀ value, relative to the total number of said targets; and/or
         (v) the percentage of the inverse cost per unit weight of said formulation encoded by said composite binary sequence, relative to the inverse cost per unit weight of the lowest cost per unit weight formulation,
         wherein when
         - an ingredient in said formulation encoded by said composite binary sequence does not exhibit a given quantitative characteristic, the percentage assigned thereto is zero; and
         - less than two ingredients were identified in said formulation in step (c13a), the score of said formulation is zero;
   **(c14)** compiling a composite binary sequences database comprising data on each of the composite binary sequences in said population of S composite binary sequences, wherein, for any given composite binary sequence, said data encodes
      - the type and number of ingredients in the formulation encoded by said composite binary sequence and their respective quantities;
      - the score of said binary sequence determined in step (c13c);
      - the cost per unit weight of the formulation encoded by said composite binary sequence; and
      - the qualitative characteristics and quantitative characteristics of the ingredients in the formulation encoded by said composite binary sequence, wherein said qualitative characteristics and said quantitative characteristics are those compiled in step (b);
   **(c15)**
      **(c15a)** duplicating the sub-population of composite binary sequences which encode a formulation having a score in the upper 50% of the population of S formulations;
      **(c15b)** randomly interchanging the terminal F% of bits in each composite binary sequence that is comprised in said duplicate sub-population of composite binary sequences with the terminal F% of bits from any other composite binary sequence in said duplicate sub-population of composite binary sequences, thus generating S/2 new composite binary sequences;
      **(c15c)** replacing the data for the sub-population of composite binary sequences in said composite binary sequence database which encode a formulation having a score in the lower 50% of the population of S formulations, with data on S/2 new composite binary sequences which were generated in step (c15b);
      **(c15d)** optionally varying the value of one randomly selected bit in each composite binary sequence comprised in
         - the sub-population of composite binary sequences which encode a formulation having a score in the upper 50% of the population of S formulations, and
         - the S/2 new composite binary sequences generated in step (c15b),
         wherein the probability that any given bit changes value is between 0 and 0.1, thus generating a new population of S composite binary sequences;
   **(c16)** repeating steps (c13) and (c14) for the new population of S composite binary sequences generated in step (c15);
   **(c17)** repeating steps (c15) and (c16) K times, wherein K is a whole number less than 1000, or until the average score of the formulations encoded by said composite binary sequence is not increased between consecutive repetitions of steps (c15) and (c16);
**(d)**
   **(d1)** selecting:
      - the random selection for combination into a formulation obtained in step (c7); or
      - the M percent of the random selections for combination into formulations obtained in step (c8) having the highest scores, wherein M is a whole number from 1 to 50, as the random selection or random selections of ingredients for combination into said at least one topical dermal formulation for cosmetic use, or
   **(d2)** selecting:
      - the composite binary sequence obtained in step (c17) encoding the formulation having the highest score; or
      - the M percent of the composite binary sequences obtained in step (c17) encoding the formulations having the highest scores, wherein M is a whole number from 1 to 50,
      as the composite binary sequence or composite binary sequences encoding said at least one topical dermal formulation for cosmetic use; and
**(e)** making said at least one topical dermal formulation for cosmetic use by mixing the ingredients:
   - comprised in the random selection or random selections, or
   - encoded by the composite binary sequence or composite binary sequences selected in step (d);
**(f)**
   **(f1)** determining the IC₅₀ and/or the EC₅₀ of said formulation or said M formulations against said target referred to in steps (c2b) or (c13b), according to the procedure in step (a); and
   **(f2)** selecting the formulation or M' percent of formulations made in step (e) having the lowest IC₅₀ and/or the EC₅₀ against said target, as determined in step (f1), wherein M' is a whole number from 1 to 20, as said at least one topical dermal formulation for cosmetic use.

Other characteristics and advantages of the present invention will be clear from the following detailed description of embodiments illustrative of its objective in relation to the attached figures.

### Brief Description of the Figures

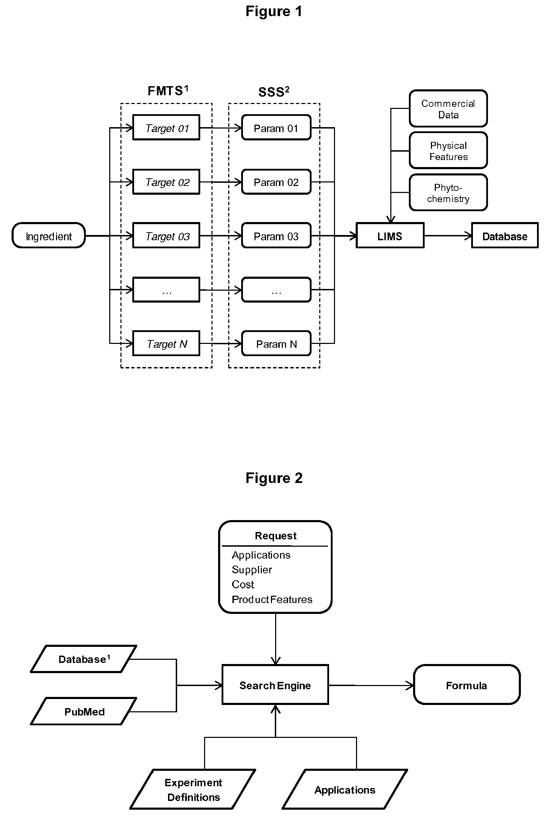

**Figure 1****.** Parametrization of experimental results regarding biological activity of dermocosmetic ingredients. The Fast Multitarget Screening (FMTS) system implemented in SimDerma consists of a panel of experimental targets which are used to test the ingredients at three different concentrations. Results may be parametrized according to a Standardized Scoring System (SSS), which, in this case, assigns value 0 to the ingredient for an specific target when it is not induced at any concentration *in vitro,* 1 when induction is observed only at the highest concentration of ingredient, 2 for induction at the intermediate concentration, but not at the lowest concentration, and 3 for ingredients inducting activity at the lowest concentration, from which value a percentage is calculated relative to the maximum value of 3. An analogous assignment of scores is applicable for ingredients with inhibitory capacity against a target, thus assigning value 0 when inhibition is not found at any of the concentrations *in vitro,* 1 when inhibition is observed only at the highest concentration of ingredient, 2 for inhibition at the intermediate concentration, but not at the lowest concentration, and 3 for ingredients inhibiting activity at the lowest concentration, from which value a percentage is calculated relative to the maximum value of 3. The resulting percentage score is set into the database by using the LIMS, with the commercial description, physicochemical features and the phytochemical analysis of the ingredient. (1): Fast Multitarget Screening. (2): Standardized Scoring System.
**Figure 2****.** Data flow in INCOS for rational design of formulas. The user query includes the main applications to be matched by the final product, commercial preferences and physicochemical or organoleptic features. Once the query has been received, system uses information from the biological activity database (1) and the public repositories (PubMed, PubChem), as a knowledge basis about the biological behavior of each ingredient under specific experimental conditions and its association to dermocosmetic applications. The search engine is an evolutionary algorithm capable of gathering optimized combinations of ingredients, minimizing production costs and maximizing the biological activity against the required experimental targets.

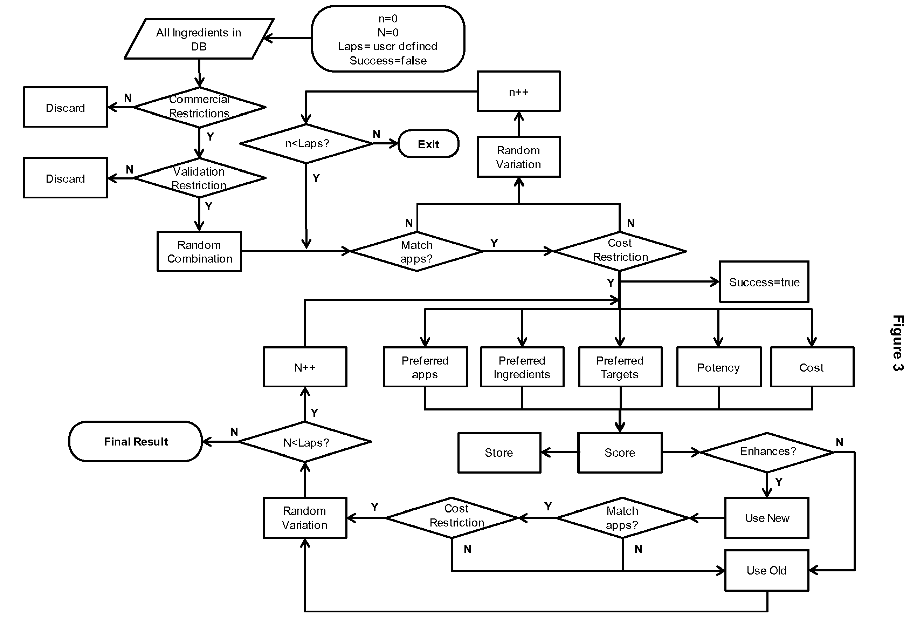

**Figure 3****.** Unified Modelling Language (UML) notation of the algorithm, which is executed in steps (c1) to (c8) of the method of the present invention in order to generate a candidate novel topical formulation for cosmetic use. First, primary filters, related to user defined specific constraints, are applied to the ingredients, thus, those not matching these constraints are discarded. Then, ingredients overcoming the primary filters are used to iteratively create random combinations until a formulation matching all the user-defined specifications is found. This formulation is used as a starting-point to get an optimized result by enhancing its composition. The number of attempts allowed to get a successful germinal formulation is set by the user and stored at variable laps. If a valid formulation is achieved, the binary variable success changes its value to true and the formulation is addressed to a second loop trying to improve its composition. The number of attempts to improve the formulation is also defined by laps. For each iteration in the enhancing loop, the system evaluates, for example, the cost of the ingredients, its activity and the uses (i.e. applicability) of the final formulation and, on this basis, a score is assigned to the formulation. Next, a random modification, consisting of, for example, a removal, addition or change of an ingredient is set to the formulation. If the new formulation scores better than its precursor and its value for success is true, said new formulation will replace the lower scored one. When the system reaches the maximum number of iterations, as given by the laps value, it will output, for example, the highest score formulation.

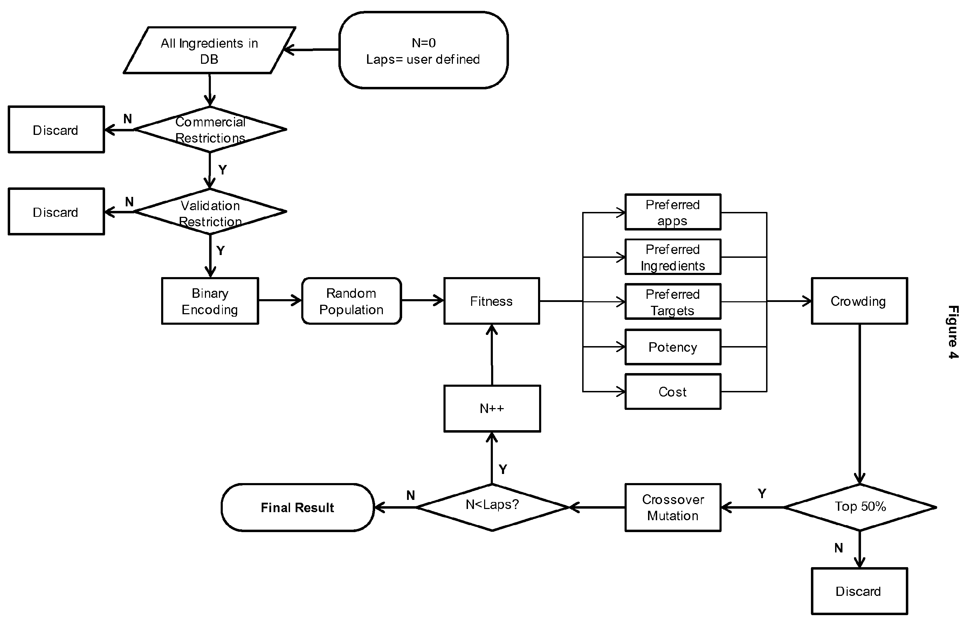

**Figure 4****.** Unified Modelling Language (UML) notation of the algorithm which is executed in steps (c10) to (c17) of the method of the present invention in order to generate a candidate formulation for a novel topical formulation for cosmetic use. Once primary filters have been applied, a binary encoded reference is assigned to the ingredients (compounds or compositions) overcoming this stage. These references are tuples of *bits,* whose size depends on the amount of ingredients to be encoded and is calculated according to the Kolmogorov algorithm complexity theory. Then, a random population of binary sequences (chromosomes) encoding lists of ingredients is created. Each chromosome size depends on the size of the tuples and the maximal amount of ingredients per formula. The population is processed as follows. First, to evaluate these formulas, chromosomes are decoded to obtain their lists of ingredients and then, they are scored attending to, for example, their cost, biological activity and applicability of the final formula and the resulting score is stored on the *fitness* variable. Second, chromosomes are ranked according to their *fitness* by the *crowding* operator, and the lowest scored half of the population is discarded. Third, chromosomes overcoming the previous stage amplify the population up to its initial size with new sequences arising from combinations among the surviving sequences by the *crossover* operator. At this point, diversification is increased in the population by the *mutation* operator, which randomly changes the value of any bit within the chromosome sequence. Last, new chromosomes are evaluated and process is iteratively repeated until, for example, the system reaches convergence or a specified number of iterations is reached. Convergence is established when the average fitness in the population remains stable during a critical number of iterations. When system reaches convergence, it outputs the formulas corresponding to, in this case, the chromosomes having best fitness.

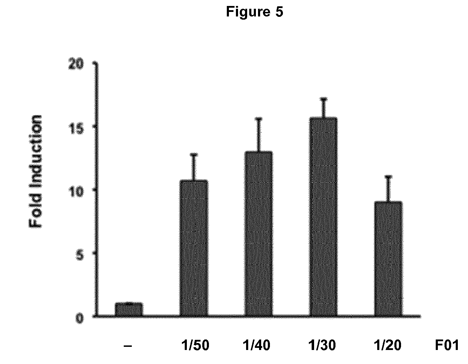

**Figure 5****.** PPARα transactivation assays in NIH-3T3 cells. The concentration of the formulation F01 (vol./vol.) is shown on the x-axis and the PPARα activation fold is shown on the y-axis. This figure shows the effect of F01 on PPARα activity. Fold activation level was calculated using the control sample (-), without the presence of any PPARα agonist or activating agent, as reference. Data are expressed as mean ± S.D. of at least three independent experiments.

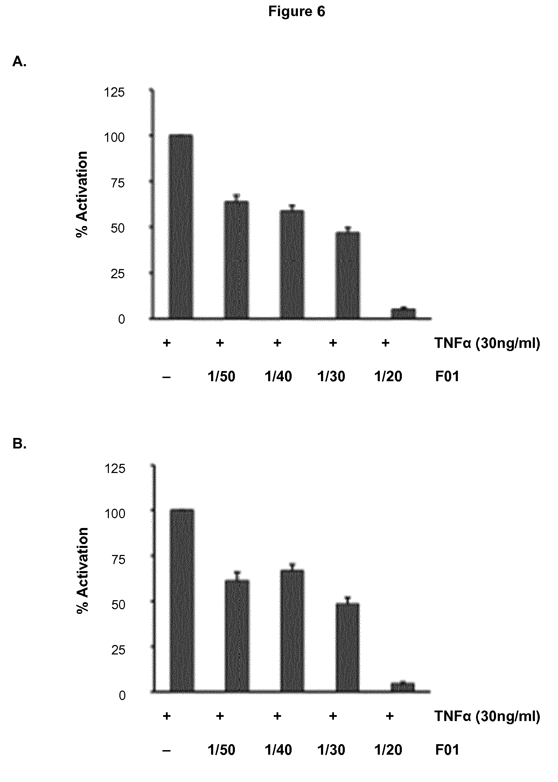

**Figure 6****.** NF-κB transactivation assays in HaCaT and NIH-3T3 cells. The concentration of the formulation F01 (vol./vol.) is shown on the x-axis and the percentage of TNFα-induced NF-κB is shown on the y-axis. **A.** NIF-3T3-KBF-Luc fibroblasts (15 x 10⁴ cells/mL) preincubated with increasing concentrations of the compounds and next treated with TNFα (30 ng/mL) for 6 hours. Luciferase activity was measured in the cell lysates and results are represented as the percentage of inhibition considering 100% the value of TNFα-induced NF-κB activation. **B.** HaCaT-KBF-Luc fibroblasts (15 x 10⁴ cells/mL) preincubated with increasing concentrations of the compounds and next treated with TNFα (30 ng/mL) for 6 hours. Luciferase activity was measured in the cell lysates and results are represented as the percentage of inhibition considering 100% the value of TNFα-induced NF-κB activation. Data are expressed as mean ± S.D. of at least three independent experiments.

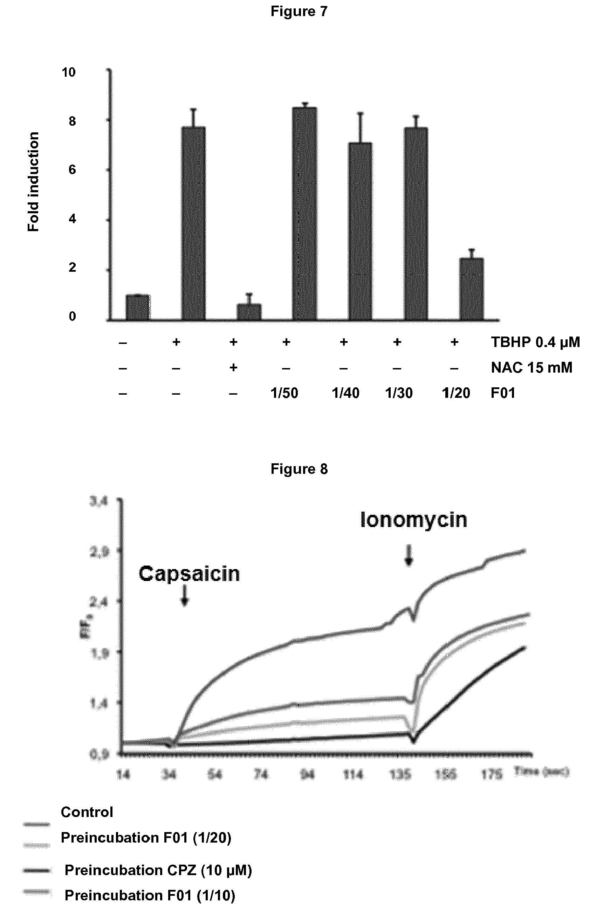

**Figure 7****.** Antioxidant activity of formulation F01. HaCaT cells were pre-incubated either with increasing concentrations of F01 (vol./vol., vol.=volume) or with *N*-acetyl-cysteine (NAC) for 15 min and then treated with 0.2 mM *tert*-butylhydroquinone (TBHQ) for 6 hours. The cells were labeled with the florescent probe H2DCF-DA (20 nM) to detect intracellular ROS. The results are represented as a fold induction of fluorescence (y-axis) taking the control sample (-) as a reference. Data are expressed as mean ± S.D. of at least three independent experiments.
**Figure 8****.** TRPV-1 antagonistic activity of formulation F01. 293T-TRPV1 cells were loaded with Fura 2 and preincubated with either F01 (1/10 and 1/20 vol./vol.) or with capsazepine (CPZ), a known TRPV-1 antagonist, for 15 seconds before the addition of 3 µM capsacin (TRPV-1 agonist). Calcium mobilization was measured by fluorescence microscopy using a Pathway BD 855 (Becton Dickinson). Vertical arrow indicates the time of compound addition. After 5 minutes of recording, ionomycin (1 µg/mL) was added to standardize results. Data are representative of at least five independent experiments.

### Detailed Description of the Invention

The present invention relates to a method for producing a topical dermal formulation for cosmetic use. In the context of the present invention a topical dermal formulation for cosmetic use is a formulation or composition (dermocosmetic product) for topical application to the skin, wherein said formulation comprises two or more ingredients (wherein each ingredient is a compound or composition) which each exhibit biological activity against at least one target and thus effect at least one cosmetic use.

Said method has its basis in the technical development of a comprehensive platform to assist in the design of topical dermal formulations for cosmetic use for topical application to the skin, comprising SimDerma hardware aimed at characterizing the biological activity of ingredients approved for cosmetic use according to the method represented by Figure 1, in combination with INCOS (Ingredients Combinatorial System) software, which comprises an interface to collect experimental results and a system for automated formulation of dermocosmetic assets oriented to specific applications, according to the method represented by Figure 2. Both tools work in a sequential manner, leading to a validated result.

In particular, the method of the present invention comprises the steps **(a)** to **(e).** Step **(a)** comprises determining the IC₅₀ of ingredients which exhibit antagonistic biological activity against at least one target, and/or determining the EC₅₀ of ingredients which exhibit agonistic biological activity against at least one target, preferably in an experimental skin model as defined herein. When said target is inhibited through antagonistic biological activity and/or when said target is activated through agonistic biological activity, it effects a cosmetic use. Therefore, by selecting at least one cosmetic use for which the topical dermal formulation produced by the present invention is intended, this defines which said at least one target and, hence, which biological activity or biological activities against said at least one target should be determined. Accordingly, for any particular cosmetic use, said at least one target of the present invention and said biological activity referred to throughout the method of the present invention are the same. Consequently, the same target(s) and the associated biological activity giving rise to a given use in a cosmetic formulation is referred to throughout, meaning that it is not the case that, for example, the concentration at the IC₅₀ against one target is used in, *e.g.* step (c2ci), but a different biological activity (*e.g*. against a different target or agonistic biological activity) is referred to in, *e.g*. step (c3).

Said cosmetic use is preferably selected from an anti-cellulitis use, an anti-aging [preferably anti-chronological (intrinsic) ageing] use, an anti-rhytid (anti-wrinkle) use, an atopic skin care use, a dark circles attenuation use, a hair growth stimulation use, a moisturizing use, a tissue refirming [preferably anti-laxity (anti-sagging)] use, a sensitive skin care use, a skin repair use, a skin whitening or sun block/sun care [preferably selected from anti-sunburn, anti-solar elastosis (anti-yellowing), anti-photosensitivity, anti-photodermatitis or anti-photo (extrinsic) ageing, more preferably anti-sunburn] use. Thus, the first step in the method of the present invention consists of the experimental assessment of the activity of any given ingredient against a pool of biological targets related to the main cosmetic use or uses of interest.

In addition, said target is selected from at least one molecule, protein, protein complex, nucleic acid or nucleic acid complex. Said target is preferably selected from at least one organic compound, protein or protein complex. In one preferred embodiment, the biological activity against a target that is analyzed in the SimDerma platform for each individual cosmetic ingredient is selected from, NF-κB activity in keratinocytes, NF-κB activity in fibroblasts, STAT3 activity, PPARγ activity, PPARα activity, induction of Nrf2 activity, inhibition of Nrf2 activity, Tcf/LET activation (Wnt pathway), total antioxidant activity, cellular antioxidant activity, glucose uptake in keratinocytes, CB1 agonistic activity, CB2 agonistic activity, TRPV-1 agonistic activity, TRPV-1 antagonistic activity, tyrosinase activity in melanocytes, melanin content in melanocytes, activation of HIF-1α pathway, inhibition of HIF-1α pathway, induction of collagen gene transcription in fibroblasts (COL1A2-Luc), induction of filaggrin gene transcription in keratinocytes, induction of cyclic AMP, inhibition of cyclic AMP, or DNA repairing activity. More preferably, the biological activity against a target is selected from NF-κB activity in keratinocytes, NF-κB activity in fibroblasts, PPARγ activity, PPARα activity, induction of Nrf2 activity, inhibition of Nrf2 activity, total antioxidant activity, cellular antioxidant activity, TRPV-1 agonistic activity or TRPV-1 antagonistic activity. Still more preferably, the biological activity against a target is selected from NF-κB activity in keratinocytes, NF-κB activity in fibroblasts, PPARα activity, cellular antioxidant activity or TRPV-1 antagonistic activity.

Step **(b)** of the present invention comprises compiling an ingredient database comprising data on each of said ingredients, wherein the data for any given ingredient encodes at least one qualitative characteristic of said ingredient and at least one quantitative characteristic of said ingredient. Said qualitative characteristic is selected from a qualitative chemical property of said given ingredient (*e.g*. presence of aromaticity), a qualitative physical property of said given ingredient (*e.g*. a qualitative phytochemical property such as ability to absorb UV-B), an organoleptic property of said given ingredient, a supplier of said given ingredient (*e.g*. a manufacturer), or a dermatological test performed using said given ingredient. On the other hand, said quantitative characteristic is selected from an IC₅₀ or EC₅₀ value of said given ingredient determined in step (a), a skin permeability value of said given ingredient, a cost per unit weight of said given ingredient, a quantitative chemical property of said given ingredient (*e.g*. number of dissociable protons), or a quantitative physical property of said given ingredient (*e.g*. pH or degree of UVA absorption). The quantitative characteristic is a value associated with the extent of said quantitative characteristic for a given ingredient or for a given ingredient at a given concentration. It may be a measured value, an assigned value based on said measured value (for example, a value of 0 may be assigned to the ingredient for a specific target when it is not induced at any of three concentrations *in vitro,* a value of 1 may be assigned when induction is observed at the highest of said three concentrations of the ingredient relative to a given pre-determined value, 2 may be assigned when induction is observed at the intermediate concentration of said three concentrations of said ingredient and 3 may be assigned when induction is observed at the lowest of said three concentrations of said ingredient) or a value which is expressed as a percentage or fraction of said measured value or said assigned value relative to a given pre-determined value.

Said qualitative characteristics and said quantitative characteristics that are compiled in said database may be user-defined, whether by virtue of the intended cosmetic use of the topical dermal formulation that is to be produced by the method of the invention, or based on the requirements of the user and/or based on the availability of said information. Thus, said characteristics may be compiled by the user, the producers of said ingredients and/or derived from the public domain (*e.g.* from databases such as PubMed or CAS).

For the purposes of the present invention, it should be noted that a chemical or physical property preferably refers to a physico-chemical property, whether qualitative (*e.g*. hygroscopic property) or quantitative (*e.g*. degree of UVA absorption). In addition, an organoleptic property refers to a property that a human experiences via the senses (*e.g*. taste or smell) while a dermatological test performed using an ingredient refers to whether or not said ingredient has already passed a standard test carried out through application of said ingredient to the skin (*e.g*. a skin contact allergy patch test or an *in vitro* UVA protection test according to ISO 24443:2012). Moreover, a skin permeability value of an ingredient refers to a measure of the absorption of said ingredient through the skin. Preferably said skin permeability is measured according to percutaneous penetration studies using *in vitro* methods [*e.g*. using static diffusion cells (Franz cells) or flow-through diffusion cells (Bronaugh cells)] or *in silico* methods [*e.g*. Quantitative Structure to Property Relationship analysis]. In said methods, the concentration of ingredient applied, the volume of the dose comprising said ingredient applied to the skin, the amount of ingredient applied per unit area of skin, the vehicle, exposure time, temperature and, in the case of *in silico* methods, the algorithm used to establish the structure to property correlation, the mean assignment error and, within the *in silico* methods, in the case of supervised machine learning methods, the size and the value range of the training sample set, the size and the value range of the testing sample set, the learning error and the training error are all specified.

Step **(c)** of the present invention comprises two alternative sets of steps, namely steps **(c1)** to **(c8)** or **(c9)** to **(c17).** Steps **(c1)** to **(c8)** correspond to the algorithm which is disclosed in Unified Modelling Language (UML) notation in Figure 3.

In particular, step **(c1)** comprises identifying those ingredients which have data encoding at least one first given qualitative characteristic of said ingredients in said ingredient database, wherein said first given qualitative characteristic is selected from a presence or absence of a given qualitative chemical property, a presence or absence of a given qualitative physical property, a presence or absence of a given organoleptic property, the provenance or not from a given supplier, or a presence or absence of passing a given dermatological test performed. Said first given qualitative characteristic is a qualitative characteristic that is selected by the user of the method of the present invention according to the requirements that the topical dermal formulation for cosmetic use must fulfil. For example, said first given qualitative characteristic may be a requirement that the topical dermal formulation only comprises ingredients that are produced by a given supplier or which have passed a given dermatological test.

Step **(c2)** comprises:
**(c2a)** making a random selection of two or more of said ingredients identified in step (c1) for combination into a formulation for said given cosmetic use; and
**(c2b)** calculating the total cost per unit weight of said formulation when all ingredients in said random selection of ingredients made in step (c2a) exhibit:
   - antagonistic biological activity (AN) against a given target or targets, wherein each given target is that which, when inhibited in an experimental skin model, effects said given cosmetic use, or
   - agonistic biological activity (AG) against a given target or targets, wherein each given target is that which, when activated in an experimental skin model, effects said given cosmetic use,
   by multiplying the cost per unit weight of each ingredient in the random selection made in step (c2a) by the concentration (C) at which it is to be combined into said formulation, wherein the concentration (C) for any given ingredient in said random selection is:
   - the IC₅₀ value of said ingredient determined in step (a) against said given target, or the maximum of the IC₅₀ values of said ingredient determined in step (a) against said given targets, when said ingredient exhibits said antagonistic biological activity (AN), or
   - the EC₅₀ value of said ingredient determined in step (a) against said given target, or the maximum of the EC₅₀ values of said ingredient determined in step (a) against said given targets, when said ingredient exhibits said agonistic biological activity (AG); and
**(c2c)** randomly varying said random selection if the formulation to be made from combining said random selection of ingredients at the concentrations (C) referred to in step (c2b):
   - comprises an ingredient that does not have at least one second given qualitative characteristic, wherein said second given qualitative characteristic is different from the at least one first given qualitative characteristic in step (c1), and wherein said second given qualitative characteristic is selected from a presence or absence of a given qualitative chemical property, a presence or absence of a given qualitative physical property, a presence or absence of a given organoleptic property, a presence or absence of a given supplier, or a presence or absence of passing a given dermatological test performed;
   - comprises an ingredient which does not exhibit the antagonistic biological activity (AN) or the agonistic biological activity (AG) referred to in step (c2b); and/or
   - does not have a total cost per unit weight calculated according to step (c2b) which is less than a given total cost per unit weight,
      by:
      **(c2ci)** randomly deselecting one of said ingredients from said random selection, provided that said random selection comprises more than two ingredients, thus decreasing the number of ingredients for combination into said formulation;
      **(c2cii)** randomly selecting a further ingredient identified in step (c1) for combination with said random selection, thus increasing the number of ingredients for combination into said formulation;
      **(c2ciii)** randomly deselecting one of said ingredients from said random selection, and randomly selecting another ingredient identified in step (c1), for combination with the remaining ingredient or ingredients of said random selection, thus maintaining constant the number of ingredients for combination into said formulation; or
      **(c2civ)** randomly deselecting a number, x, of said ingredients from said random selection, and randomly selecting a further x ingredients from any of the random selections generated in step (c5), for combination with the remaining ingredient or ingredients of said random selection, provided that said random selection comprises more than (x + 1) ingredients, thus maintaining constant the number of ingredients for combination into said formulation; and
**(c2d)** repeating steps (c2b) and (c2c) n times, wherein n is a whole number less than 100, until:
   - all ingredients comprised in said random selection of ingredients have said at least one second given qualitative characteristic,
   - all ingredients comprised in said random selection of ingredients exhibit the antagonistic biological activity (AN) or the agonistic biological activity (AG) referred to in step (c2b), and
   - said formulation to be made from combining said random selection of ingredients has a total cost per unit weight calculated according to step (c2b) which is less than said given total cost per unit weight.

The experimental skin model may be any skin model used for clinical testing of products to be applied to the external skin surface. In a preferred embodiment of the present invention, the experimental skin model is an *in vitro* skin cell model comprising at least one cell type selected from keratinocytes, melanocytes, Langerhans cells, dermal fibroblasts, dermal dendritic cells, skin resident T-cells or endothelial cells.

Given that the random selection referred to throughout the present invention is a random selection of two or more ingredients identified in step (c1), more preferably a random selection of between 2 and 20 ingredients identified in step (c1), said random selection therefore comprises multiple (2 or more) different random elections of ingredients, each election being from the same list of ingredients. The elected ingredients comprised in each of said random selections is for combination into a formulation at the respective IC₅₀ or EC₅₀ concentrations determined in step (a), whereby whether combination at the IC₅₀ concentration or at the EC₅₀ concentration is used is determined by the at least one intended cosmetic use of the topical dermal formulation to be produced by the method of the invention, given that said at least one cosmetic use defines which said at least one target and, hence, whether agonistic or antagonistic biological activity or biological activity is required.

In addition, by analogy with the first given qualitative characteristic, said second given qualitative characteristic is a qualitative characteristic other than a first given qualitative characteristic that is selected by the user of the method of the present invention according to the requirements that the topical dermal formulation for cosmetic use must fulfil. For example, if it is required that the topical dermal formulation only comprises ingredients that have passed a given dermatological test or that are produced by a given supplier, said second given qualitative characteristic may be that said ingredients must show a given organoleptic property. In addition, said ingredients must have a given cost per unit weight that is less than a user-defined amount.

Step (c2d) comprises synchronously repeating steps (c2b) and (c2d) n times to make n random selections of two or more ingredients identified in step (c1) for combination into n respective formulations, wherein n is a whole number less than 100 by default, or a user-defined number of variants. Preferably n is a whole number between 2 and 50, more preferably a whole number between 5 and 20.

Step **(c3)** comprises determining the score of said random selection of two or more ingredients for combination into said formulation, wherein said score is determined by:
- averaging the mean score of each of the ingredients comprised in said random selection, wherein said mean score of any given ingredient is determined by averaging the percentage of a value of at least one quantitative characteristic of said given ingredient at the concentration (C) referred to in step (c2b), relative to a corresponding predetermined value of said at least one quantitative characteristic, and/or determining the percentage of a value of at least one quantitative characteristic of said formulation relative to a corresponding predetermined value of said at least one quantitative characteristic; and/or
- averaging the mean score of each of the ingredients comprised in said random selection, wherein said mean score of any given ingredient is determined by averaging the percentage of an inverse value of at least one quantitative characteristic of said given ingredient at the concentrations (C) referred to in step (c2b), relative to an inverse of a corresponding predetermined value of said at least one quantitative characteristic, and/or determining the percentage of an inverse value of at least one quantitative characteristic of said formulation relative to an inverse of a corresponding predetermined value of said at least one quantitative characteristic;
wherein said quantitative characteristic of each ingredient is selected from an IC₅₀ or EC₅₀ value of said ingredient determined in step (a), a skin permeability value of said ingredient, a cost per unit weight of said ingredient, a quantitative chemical property of said ingredient, or a quantitative physical property of said ingredient, and
wherein said quantitative characteristic of said formulation is selected from a total cost per unit weight of said formulation, a total number of ingredients comprised in said formulation, an IC₅₀ or EC₅₀ value of said formulation against at least one target, a quantitative chemical property of said formulation, or a quantitative physical property of said formulation, and
wherein when an ingredient in said random selection of two or more ingredients does not exhibit a given quantitative characteristic, the percentage assigned thereto is zero.
Please note that in the present invention an inverse value (or inverse of said value) refers to (said value)⁻¹ [*i.e*. 1/(said value)].

Each quantitative characteristic used in scoring, as determined in step (c3) but also that determined in steps (c5c) and (c13c), below, is a value associated with the extent of said quantitative characteristic for a given ingredient or for a given ingredient at a given concentration, or for a given formulation. As before, it may be a measured value, an assigned value based on said measured value (for example, a value of 0 may be assigned to the ingredient for a specific target when it is not induced at any of three concentrations *in vitro,* a value of 1 may be assigned when induction is observed at the highest of said three concentrations of the ingredient relative to a given pre-determined value, a value of 2 may be assigned when induction is observed at the intermediate concentration of said three concentrations of said ingredient and a value of 3 may be assigned when induction is observed at the lowest of said three concentrations of said ingredient) or a value which is expressed as a percentage or fraction of said measured value or said assigned value relative to a given pre-determined value. Preferably, each quantitative characteristic used in scoring is a value which is expressed as a percentage or fraction of a measured value or an assigned value based on said measured value, relative to a given pre-determined value. In the present invention, a predetermined value of a quantitative characteristic refers to a value that is predetermined (or preestablished) as a reference value against which the corresponding measured value of at least one quantitative characteristic of a given ingredient or formulation may be compared.

In a preferred embodiment of the method of the present invention, the mean score for any given ingredient for combination into a formulation, as determined in step (c3), but also that determined in steps (c5c) and (c13c), below, is preferably determined by averaging the following parameters:
(i) the percentage of:
   - an assigned value based on the IC₅₀ value of said given ingredient in said formulation referred to in steps (c2a), (c5a) or (c13a) against at least one given target, relative to a maximum possible assigned value, wherein a value of 0 may be assigned to said given ingredient against a given target when it is not inhibited at any concentration *in vitro,* a value of 1 may be assigned to said given ingredient when inhibition is observed at a concentration falling above the upper quartile of the IC₅₀ values for each ingredient in all of said n formulations against said at least one given target; a value of 2 may be assigned to said given ingredient when inhibition is observed at a concentration falling between the upper and lower quartiles of the IC₅₀ values for each ingredient in all of said n formulations against said at least one given target, and a value of 3 (the maximum possible assigned value) may be assigned when inhibition is observed at a concentration falling below the lower quartile of the IC₅₀ values for each ingredient in all of said n formulations against said at least one given target; or
   - the inverse of the IC₅₀ value of said given ingredient in said formulation referred to in steps (c2a), (c5a) or (c13a) against at least one given target, relative to the inverse of the maximum IC₅₀ value against said at least one given target for an ingredient in any of said n formulations;
(ii) the percentage of:
   - an assigned value based on the EC₅₀ value of said given ingredient in said formulation referred to in steps (c2a), (c5a) or (c13a) against at least one given target, relative to a maximum possible assigned value, wherein a value of 0 may be assigned to said given ingredient against a given target when it is not activated/induced at any concentration *in vitro,* a value of 1 may be assigned to said given ingredient when activation/induction is observed at a concentration falling above the upper quartile of the EC₅₀ values for each ingredient in all of said n formulations against said at least one given target; a value of 2 may be assigned to said given ingredient when activation/induction is observed at a concentration falling between the upper and lower quartiles of the EC₅₀ values for each ingredient in all of said n formulations against said at least one given target, and a value of 3 (the maximum possible assigned value) may be assigned when activation/induction is observed at a concentration falling below the lower quartile of the EC₅₀ values for an ingredient in any of said n formulations against said at least one given target; or
   - the inverse of the mean EC₅₀ value of said given ingredient in said formulation referred to in steps (c2a), (c5a) or (c13a) against at least one given target, relative to the inverse of the maximum EC₅₀ value against said at least one given target for an ingredient in any of said n formulations;
(iii) the percentage of the inverse cost per unit weight of said ingredient, relative to the inverse cost per unit weight of the highest cost per unit weight ingredient in any of said n formulations; and/or
(iv) the percentage of the skin permeability value of said ingredient, relative to the maximum skin permeability value for an ingredient in any of said n formulations.
At the same time, the score for any given formulation, as determined in step (c3), but also that determined in steps (c5c) and (c13c), below, is preferably determined by calculating:
(i) the percentage of the number of cosmetic uses of said formulation, relative to the greatest number of cosmetic uses of said n formulations;
(ii) the percentage of the number of targeted cosmetic uses of said formulation relative to the maximum number of targeted cosmetic uses;
(iii) the percentage of the number of ingredients identified in step (c1) or step (c9) which are comprised in said formulation, relative to the maximum number of ingredients permissible in said formulation;
(iv) the percentage of the number of targets against which said formulation exhibits an IC₅₀ or EC₅₀ value, relative to the total number of said targets; and/or
(v) the percentage of the inverse cost per unit weight of said formulation, relative to the inverse cost per unit weight of the lowest cost per unit weight formulation.

In another preferred embodiment of the method of the present invention each of said parameters used to determine the scores in steps (c3), (c5c) and (c13c) is optionally independently weighted by a value between 0 and 1. In a more preferred embodiment of the foregoing, each parameter is weighted equally by a value between 0.1 and 1. Weighting is carried out by multiplying each parameter by said given weight, wherein the resulting weighted parameters are averaged to calculate said score. In a further preferred embodiment of the method of the present invention, each parameter used to determine the scores in steps (c3), (c5c) and (c13c) is weighted equally.

Step **(c4)** comprises compiling a formulation database comprising data on said random selection made in steps (c2), wherein said data encodes:
- the type and number of ingredients comprised in said random selection and their respective quantities;
- the score of said random selection determined in step (c3);
- the total cost per unit weight of said formulation to be made from combining said random selection of ingredients; and
- the qualitative characteristics and quantitative characteristics of the ingredients comprised therein, wherein said qualitative characteristics and said quantitative characteristics are those compiled in step (b).

Step **(c5)** comprises:
**(c5a)** randomly varying said random selection for which data is compiled in said formulation database to generate a new random selection for combination into a new formulation for said given cosmetic use by:
   **(c5ai)** randomly deselecting one of said ingredients from said random selection, provided that said given random selection comprises more than two ingredients, thus decreasing the number of ingredients for combination into said formulation;
   **(c5aii)** randomly selecting a further ingredient identified in step (c1) for combination with said random selection, thus increasing the number of ingredients for combination into said formulation;
   **(c5aiii)** randomly deselecting one of said ingredients from said random selection, and randomly selecting a further ingredient identified in step (c1), for combination with the remaining ingredient or ingredients of said random selection, thus maintaining constant the number of ingredients for combination into said formulation; or
   **(c5aiv)** randomly deselecting a number, x, of said ingredients from said random selection, and randomly selecting a further x ingredients from any of the random selections generated in step (c8), for combination with the remaining ingredient or ingredients of said random selection, provided that said random selection comprises more than (x + 1) ingredients, thus maintaining constant the number of ingredients for combination into said formulation;
**(c5b)** calculating the total cost per unit weight of said new formulation according to step (c2b), provided that all ingredients comprised in said new random selection of two or more ingredients obtained in step (c5a) exhibit the antagonistic biological activity (AN) or the agonistic biological activity (AG) referred to in step (c2b); and
**(c5c)** determining the score of said new random selection of two or more ingredients obtained in step (c5a) for combination into said new formulation according to step (c3). The value of x is determined by the number of ingredients in said random selection, whereby at most, x = (the number of ingredients in said random selection - 1). Accordingly, given that the random selection of two or more ingredients is, for example, preferably between 2 and 10 compounds, x must preferably be a number between 1 and 9.

Step **(c6)** comprises replacing the data on said random selection in said formulation database with the corresponding data on said new random selection if:
- the score of said new random selection determined in step (c5c) is greater than the score of said random selection in said formulation database;
- said new random selection comprises an ingredient that has the at least one second given qualitative characteristic referred to in step (c2c);
- said new random selection comprises an ingredient which exhibits the antagonistic biological activity (AN) or the agonistic biological activity (AG) referred to in step (c2b); and/or
- said new random selection has a total cost per unit weight calculated according to step (c5b) which is less than said given total cost per unit weight.

Step **(c7)** comprises repeating steps (c5) and (c6) N times, wherein N is a whole number less than 100 by default or a user-defined number of iterations, preferably a whole number between 2 and 50, more preferably a whole number between 5 and 20. In a further preferred embodiment, N = n = 50 or, still more preferably, N = n = 20.

Step **(c8)** comprises optionally repeating steps (c2) to (c7) S times to afford S random selections of two or more of said ingredients for combination into S respective formulations, wherein S is a whole number less than 1000, preferably less than 500, more preferably less than 100.

Steps **(c1)** to **(c8)** were conceived as a light algorithm to work with databases including a low number of compounds and/or compositions, usually below 200 entries, as per step (b). In an alternative embodiment, a list of said compounds and/or compositions is not encoded in step (b), but instead directly stored at the memory stack as object instances including all their features, which allows the method of the present invention to be performed by using the internal methods of the class used to simulate the ingredients. Subsequently, said compounds and/or compositions are subjected to a filtering process, which allows those compounds and/or compositions that, for example, are not dermatologically proven or that do not fit some commercial requirements to be discarded, as per step (c1). These filters, which are applied to the compounds and/or compositions before formulations have begun to be generated, are called primary filters. Step (c2) of the method of the present invention therefore starts by creating random selections of compounds and/or compositions which are also passed through further primary filters until one random selection fitting a formulation having, for example, a given total cost per unit weight, is identified. This random selection corresponds to "successful" formulations, which are so-called "germinal formulations", in that they are used as starting points from which new enhanced formulations are obtained. The number of attempts, n, to obtain a germinal formulation is set by the user and is stored at the so-called "laps" variable. New enhanced formulations are subsequently obtained from each germinal formulation by, for example, replacing, removing or adding some ingredients over a user-defined number of iterations, as per step (c5). For each iteration, the resulting new random selection corresponding to a new formulation is scored and replaces the previous formula if it has a higher score and overcomes all the primary filters, as per step (c6). If a random selection corresponding to a new formulation having higher score is obtained, the random selection corresponding to said new formulation is addressed to a further iteration(s) in order to improve its composition. The number of attempts to improve the formulation is also defined by laps, whereby after a number of iterations set by the user, the method of the present invention provides the highest scored random selection or a percentage of random selections, M, having the highest score, for making into a formulation or formulations.

On the other hand, steps **(c9)** to **(c17)** correspond to the algorithm disclosed in UML notation in Figure 4.

In particular, step **(c9)** comprises identifying those ingredients which have data encoding at least one given qualitative characteristic of said ingredients in said ingredient database wherein, for a given ingredient, said given qualitative characteristic is selected from a presence or absence of a given qualitative chemical property of said given ingredient, a presence or absence of a given qualitative physical property of said given ingredient, a presence or absence of a given organoleptic property of said given ingredient or the provenance or not from a given supplier of said given ingredient;

Each given qualitative characteristic is a characteristic that is selected by the user of the method of the present invention according to the requirements that the topical dermal formulation for cosmetic use must fulfil. For example, said given qualitative characteristic may be a requirement that the topical dermal formulation only comprises ingredients that have passed a given dermatological test or that are produced by a given supplier.

Step **(c10)** comprises generating 2^{T} binary sequences, wherein each binary sequence reads from left to right and comprises a tuple, T, of bits, wherein T is calculated by rounding up to the nearest whole number the result of log₂(NI), wherein NI is the number of ingredients identified in step (c9), wherein each bit is additionally assigned a value between 0 and 0.1 corresponding to the probability that said bit changes. A tuple of bits is a finite, ordered list of bits (each bit having a value of 0 or 1). The probability that said bit changes refers to the probability that said bit changes when seeking to achieve an improved score. In a preferred embodiment, each bit is additionally assigned a value of 0.02 corresponding to the probability that said bit changes.

Step **(c11)** comprises assigning each of the ingredients identified in step (c9) one of the binary sequences generated in step (c10). Unless NI = 2^{T}, at least one binary sequence will not be assigned to an ingredient.

Step **(c12)** comprises generating a population of S composite binary sequences, wherein each composite binary sequence reads from left to right and comprises P binary sequences reading from left to right and joined in linear sequence, and wherein
- each of the P binary sequences is randomly selected from the 2^{T} binary sequences generated in step (c10);
- S is a whole number less than 1000; and
- P is a given whole number between 2 and 20 which refers to the maximum possible number of ingredients in said formulation for said given cosmetic use. Preferably S is a whole number less than 500, more preferably less than 100, and P is a whole number between 2 and 10, more preferably between 2 and 8.

Step **(c13)** comprises, for each composite binary sequence:
**(c13a)** identifying which of the ingredients identified in step (c9) are encoded by the P binary sequences comprised therein for combination into a formulation encoded by said composite binary sequence;
**(c13b)** calculating the total cost per unit weight of the formulation encoded by said composite binary sequence, provided that at least two ingredients were identified in step (c13a) and all ingredients identified in step (c13a) exhibit:
   - antagonistic biological activity (AN) against a given target or targets, wherein each given target is that which, when inhibited in an experimental skin model, effects said given cosmetic use, or
   - agonistic biological activity (AG) against a given target or targets, wherein each given target is that which, when activated in an experimental skin model, effects said given cosmetic use,
   by multiplying the cost per unit weight of each ingredient identified in step (c13a) by the concentration (C) at which it is to be combined into the formulation encoded by said composite binary sequence, wherein the concentration (C) for any given ingredient identified in step (c13a) is:
   - the IC₅₀ value of said given ingredient as determined in step (a) against said given target, or the maximum of the IC₅₀ values of said ingredient determined in step (a) against said given targets, when said ingredient exhibits said antagonistic biological activity (AN), or
   - the EC₅₀ value of said given ingredient as determined in step (a) against said given target, or the maximum of the EC₅₀ values of said ingredient determined in step (a) against said given targets, when said ingredient exhibits said agonistic biological activity (AG); and
**(c13c)** determining the score of the formulation encoded by said composite binary sequence, wherein said score is determined by:
   - averaging the mean score of each of the ingredients identified in step (c13a), wherein said mean score of any given ingredient is determined by averaging the percentage of a value of at least one quantitative characteristic of said given ingredient relative to a corresponding predetermined value of said at least one quantitative characteristic, and/or determining the percentage of a value of at least one quantitative characteristic of the formulation encoded by said composite binary sequence relative to a corresponding predetermined value of said at least one quantitative characteristic; and/or
   - averaging the mean score of each of the ingredients identified in step (c13a), wherein said mean score of any given ingredient is determined by averaging the percentage of an inverse value of at least one quantitative characteristic of said given ingredient relative to an inverse of a corresponding predetermined value of said at least one quantitative characteristic, and/or determining the percentage of an inverse value of at least one quantitative characteristic of the formulation encoded by said composite binary sequence relative to an inverse of a corresponding predetermined value of said at least one quantitative characteristic;
   wherein said quantitative characteristic of each ingredient identified in step (c13a) is selected from an IC₅₀ or EC₅₀ value of said ingredient determined in step (a), a skin permeability value of said ingredient, a cost per unit weight of said ingredient, a quantitative chemical property of said ingredient or a quantitative physical property of said ingredient, and
   wherein said quantitative characteristic of the formulation encoded by said composite binary sequence is selected from a cost per unit weight of said formulation, a total number of ingredients comprised in said formulation, an IC₅₀ or EC₅₀ value of said formulation against at least one target, a quantitative chemical property of said formulation, or a quantitative physical property of said formulation and
   wherein when:
   - an ingredient identified in step (c13a) does not exhibit a given quantitative characteristic, the percentage assigned thereto is zero; and
   - less than two ingredients were identified in step (c13a) the score of the formulation encoded by said composite binary sequence is zero.

As mentioned above in relation to step (c3), each quantitative characteristic used in scoring is a value associated with the extent of said quantitative characteristic for a given ingredient or for a given ingredient at a given concentration, or for a given formulation. As before, it may be a measured value, an assigned value based on said measured value (for example, a value of 0 may be assigned to the ingredient for a specific target when it is not induced at any of three concentrations *in vitro,* a value of 1 may be assigned when induction is observed at the highest of said three concentrations of the ingredient relative to a given pre-determined value, a value of 2 may be assigned when induction is observed at the intermediate concentration of said three concentrations of said ingredient and a value of 3 may be assigned when induction is observed at the lowest of said three concentrations of said ingredient) or a value which is expressed as a percentage or fraction of said measured value or said assigned value relative to a given pre-determined value. Preferably, each quantitative characteristic used in scoring is a value which is expressed as a percentage or fraction of a measured value or an assigned value based on said measured value, relative to a given pre-determined value.

In a preferred embodiment of the method of the present invention, the mean score for any given ingredient for combination into a formulation, as determined in step (c13c), is preferably determined by averaging the following parameters:
(i) the percentage of:
   - an assigned value based on the IC₅₀ value of said given ingredient in said formulation referred to in step (c13a) against at least one given target, relative to a maximum possible assigned value, wherein a value of 0 may be assigned to said given ingredient against a given target when it is not inhibited at any concentration *in vitro,* a value of 1 may be assigned to said given ingredient when inhibition is observed at a concentration falling above the upper quartile of the IC₅₀ values for each ingredient in all of said n formulations against said at least one given target; a value of 2 may be assigned to said given ingredient when inhibition is observed at a concentration falling between the upper and lower quartiles of the IC₅₀ values for each ingredient in all of said n formulations against said at least one given target, and a value of 3 (the maximum possible assigned value) may be assigned when inhibition is observed at a concentration falling below the lower quartile of the IC₅₀ values for each ingredient in all of said n formulations against said at least one given target; or
   - the inverse of the IC₅₀ value of said given ingredient in said formulation referred to in step (c13a) against at least one given target, relative to the inverse of the maximum IC₅₀ value against said at least one given target for an ingredient in any of said n formulations;
(ii) the percentage of:
   - an assigned value based on the EC₅₀ value of said given ingredient in said formulation referred to in step (c13a) against at least one given target, relative to a maximum possible assigned value, wherein a value of 0 may be assigned to said given ingredient against a given target when it is not activated/induced at any concentration *in vitro,* a value of 1 may be assigned to said given ingredient when activation/induction is observed at a concentration falling above the upper quartile of the EC₅₀ values for each ingredient in all of said n formulations against said at least one given target; a value of 2 may be assigned to said given ingredient when activation/induction is observed at a concentration falling between the upper and lower quartiles of the EC₅₀ values for each ingredient in all of said n formulations against said at least one given target, and a value of 3 (the maximum possible assigned value) may be assigned when activation/induction is observed at a concentration falling below the lower quartile of the EC₅₀ values for an ingredient in any of said n formulations against said at least one given target; or
   - the inverse of the mean EC₅₀ value of said given ingredient in said formulation referred to in step (c13a) against at least one given target, relative to the inverse of the maximum EC₅₀ value against said at least one given target for an ingredient in any of said n formulations;
(iii) the percentage of the inverse cost per unit weight of said ingredient, relative to the inverse cost per unit weight of the highest cost per unit weight ingredient in any of said n formulations; and/or
(iv) the percentage of the skin permeability value of said ingredient, relative to the maximum skin permeability value for an ingredient in any of said n formulations.

At the same time, the score for any given formulation, as determined in step (c13c) is preferably determined by calculating:
(i) the percentage of the number of cosmetic uses of said formulation, relative to the greatest number of cosmetic uses of said n formulations;
(ii) the percentage of the number of targeted cosmetic uses of said formulation relative to the maximum number of targeted cosmetic uses;
(iii) the percentage of the number of ingredients identified in step (c9) which are comprised in said formulation, relative to the maximum number of ingredients permissible in said formulation;
(iv) the percentage of the number of targets against which said formulation exhibits an IC₅₀ or EC₅₀ value, relative to the total number of said targets; and/or
(v) the percentage of the inverse cost per unit weight of said formulation, relative to the inverse cost per unit weight of the lowest cost per unit weight formulation.

In another preferred embodiment of the method of the present invention each of said parameters used to determine the scores in step (c13c) is optionally independently weighted by a value between 0 and 1. In a more preferred embodiment of the foregoing, each parameter is weighted equally by a value between 0.1 and 1. Weighting is carried out by multiplying each parameter by said given weight, wherein the resulting weighted parameters are averaged to calculate said score. In a further preferred embodiment of the method of the present invention, each parameter used to determine the scores in step (c13c) is weighted equally.

Step **(c14)** comprises compiling a composite binary sequences database comprising data on each of the composite binary sequences in said population of S composite binary sequences, wherein, for any given composite binary sequence, said data encodes
- the type and number of ingredients in the formulation encoded by said composite binary sequence and their respective quantities;
- the score of said binary sequence determined in step (c13c);
- the cost per unit weight of the formulation encoded by said composite binary sequence; and
- the qualitative characteristics and quantitative characteristics of the ingredients in the formulation encoded by said composite binary sequence, wherein said qualitative characteristics and said quantitative characteristics are those compiled in step (b). Step (c14) is analogous to step (c4).

Step **(c15)** comprises:
**(c15a)** duplicating the sub-population of composite binary sequences which encode a formulation having a score in the upper 50% of the population of S formulations;
**(c15b)** randomly interchanging the terminal F% of bits in each composite binary sequence that is comprised in said duplicate sub-population of composite binary sequences with the terminal F% of bits from any other composite binary sequence in said duplicate sub-population of composite binary sequences, thus generating S/2 new composite binary sequences;
**(c15c)** replacing the data for the sub-population of composite binary sequences in said composite binary sequence database which encode a formulation having a score in the lower 50% of the population of S formulations, with data on S/2 new composite binary sequences which were generated in step (c15b);
**(c15d)** optionally varying the value of one randomly selected bit in each composite binary sequence comprised in
   - the sub-population of composite binary sequences which encode a formulation having a score in the upper 50% of the population of S formulations, and
   - the S/2 new composite binary sequences generated in step (c15b),
wherein the probability that any given bit changes value is between 0 and 0.1, thus generating a new population of S composite binary sequences. S is as defined previously. The terminal F% of bits may be between the terminal 100% and the terminal 0% of bits (*i.e*. all, a terminal part or none of the bits), preferably between the terminal 50% and terminal 1% of bits, more preferably between the terminal 10% and 1% of bits.

Thus, step (c15) comprises a "selection of the fittest", whereby the lower-scored half of the population of n random selections (and the data related thereto) is iteratively replaced by a set of "genetically varied" random selections (and the data related thereto) derived from the upper-scored half of the population of n random selections by varying the value of any single bit in each of the binary references encoding the data for each random selection in said upper-scored half of the population of n random selections.

Step **(c16)** comprises repeating steps (c13) and (c14) for the new population of S composite binary sequences generated in step (c15).

Step **(c17)** comprises repeating steps (c15) and (c16) K times, wherein K is a whole number less than 1000 by default, or until the average score of the formulations encoded by said composite binary sequence is not increased between consecutive repetitions of steps (c15) and (c16). K is preferably a whole number less than 500, more preferably less than 100.

Thus, steps **(c9)** to **(c17)** form a genetic algorithm (GA). This is a stochastic search method based on the natural evolution of the biological species. It is applicable to optimization problems when the final solution accepts some tolerance on its composition. Since the rational design of formulations is a typical optimization problem with tolerance in its composition, GAs may be used to solve this. Thus, in these steps, the lists of compounds and/or compositions are encoded as binary sequences that are optimized to match all the requested cosmetic uses over the iterative stages. Said algorithm is illustrated by Figure 4 wherein, once primary filters have been applied as per step (c9), a binary encoded reference is assigned to the compounds and/or compositions overcoming this stage, as per steps (c10) and (c11). Said binary encoded references are tuples of bits whose size depend on the amount of ingredients to be encoded and are calculated according to the Kolmogorov algorithm complexity theory. Then, a random population of binary sequences (chromosomes) encoding lists of compounds and/or compositions is created, as per step (c12). The chromosome size depends on the size of the tuples and the maximal amount of compounds and/or compositions per formulation. First, to evaluate these formulations, chromosomes are decoded to obtain their lists of ingredients and then, they are scored, attending to their cost, biological activity and applicability of the final formulation, as per step (c13), and the resulting score is stored in the database of step (c14) as the fitness variable. Second, chromosomes are ranked according to their fitness by the crowding operator, and the lowest scored half of the population is discarded, whereby chromosomes overcoming the previous stage amplify the population up to its initial size with new sequences arising from combinations among the surviving sequences by the crossover operator, as per step (c15). At this point, diversification is increased in the population by the mutation operator, which randomly changes the value of any bit within the chromosome sequence. Last, new chromosomes are evaluated and the process is iteratively repeated as per steps (c16) and (c17), preferably until the system reaches convergence. Convergence may be established when the average fitness in the population remains stable during a critical number of iterations, following which the binary sequence(s) encoding the formula or formulas having the greatest score is output.

Thus, the method of the present invention may use one of two alternative algorithms to create novel formulations:
(i) a light method based on a stochastic search system that starts from a random selection of ingredients and seeks to improve the activity of the corresponding formulation by replacing, adding and removing ingredients (*cf*. Figure 3); or
(ii) a genetic algorithm that starts from a random population of formulations which are optimized by iteration according to their intended cosmetic use (*cf.* Figure 4).

Besides the iterative methodology, other approaches may be used to optimize the random selection of cosmetic ingredients to match specific cosmetic uses. In particular, Simulated Annealing is a stochastic method. It is based on statistical mechanics and its goal is to find a global minimum within a system showing several local minima. A search engine based on Simulated Annealing for design of cosmetic formulations may be implemented as follows. First, a random list of ingredients is encoded in a binary sequence in a similar way as in the method of steps (c9) to (c17). Second, a probability (σ) of changing a single bit value is established. Thus, every bit shows a probability σ, greater than 0 and less than 1, to change its value. Third, a binary sequence is iteratively subjected to random changes according to the σ probability. In the first iteration, σ value is established in the upper half of the range from 0 to 1 (*i.e.* 0,1), thus the encoded formulation will undergo considerable changes. In next steps, the σ value will decrease in an exponential way, leading to a reduced rate of change for each new iteration over the previous one. Fourth, the corresponding new formulation encoded by the new binary sequence is scored as in the (evolutive) algorithms of steps (c9) to (c17) and, if it has an improved score over its precursor, said new formula and, hence, the new binary sequence, will replace the old one. Lastly, when the maximal number of iterations is exhausted, or when the score is not improved over a given number of iterations, the method will output the binary sequence encoding the formulation having the greatest score.

Step (d) of the present invention comprises:
**(d1)** selecting:
   - the random selection for combination into a formulation obtained in step (c7); or
   - the M percent of the random selections for combination into formulations obtained in step (c8) having the highest scores, wherein M is a whole number from 1 to 50,
   as the random selection or random selections of ingredients for combination into said at least one topical dermal formulation for cosmetic use, or
**(d2)** selecting:
   - the composite binary sequence obtained in step (c17) encoding the formulation having the highest score; or
   - the M percent of the composite binary sequences obtained in step (c17) encoding the formulations having the highest scores, wherein M is a whole number from 1 to 50,
   as the composite binary sequence or composite binary sequences encoding said at least one topical dermal formulation for cosmetic use. More preferably, M is a whole number from 1 to 20. Thus, in one embodiment, the output of step (d) is a random selection or composite binary sequence corresponding to a formulation, or a set of random selections or a set of composite binary sequences, corresponding to a set of candidate formulations for the topical dermal formulation for cosmetic use.

Step **(e)** of the present invention comprises making said at least one topical dermal formulation for cosmetic use. This is achieved by mixing the ingredients:
- comprised in the random selection or random selections, or
- encoded by the composite binary sequence or composite binary sequences selected in step (d)

Optionally, each of said topical dermal formulations for cosmetic use may also be combined with a carrier or excipient. Thus, the method of the present invention is used to iteratively create random combinations until a formulation matching all the user-defined specifications is found.

In one preferred embodiment of the present invention:
(i) said at least one topical dermal formulation for cosmetic use is between one and 100 topical dermal formulations for cosmetic use, more preferably between 1 and 10 topical dermal formulations for cosmetic use;
(ii) said at least one target is between 1 and 100 targets, more preferably between 1 and 10 targets;
(iii) said at least one target is selected from an organic compound, protein or protein complex;
(iv) said at least one cosmetic use is between 1 and 20 cosmetic uses, more preferably between 1 and 10 cosmetic uses, still more preferably between 1 and 5 cosmetic uses;
(v) said at least one qualitative characteristic of said ingredient is between 1 and 20 qualitative characteristics, more preferably between 1 and 10 qualitative characteristics;
(vi) said at least one quantitative characteristic of said ingredient is between 1 and 20 quantitative characteristics, more preferably between 1 and 10 quantitative characteristics;
(vii) said at least one quantitative characteristic of said given formulation is between 1 and 20 quantitative characteristics, more preferably between 1 and 10 quantitative characteristics; and
(viii) said random selection of two or more ingredients is between 2 and 10 ingredients, more preferably between 2 and 8 compounds; still more preferably between 3 and 5 ingredients. Thus, x must preferably be a number between 1 and 9, more preferably between 1 and 7; still more preferably between 2 and 4.

In a more preferred embodiment of the foregoing, M is a whole number from 1 to 20, S is less than 100, P is less than or equal to 10, the terminal F% of bits is between the terminal 50% and terminal 1% of bits, n is 50 and N is 50.

In a preferred embodiment of the present invention, the method comprises a further step (f), wherein step (f) comprises:
(f1) determining the IC₅₀ and/or the EC₅₀ of said formulation or said M formulations against said target referred to in steps (c2b) or (c13b), according to the procedure in step (a); and
(f2) selecting the formulation or M' percent of formulations made in step (e) having the lowest IC₅₀ and/or the EC₅₀ against said target, as determined in step (f1), wherein M' is a whole number from 1 to 50, more preferably a whole number from 1 to 20, as said at least one topical dermal formulation for cosmetic use.

Thus, in a yet more preferred embodiment of any of the foregoing, M' is a whole number from 1 to 20, S is less than 100, P is less than or equal to 8, the terminal F% of bits is between the terminal 10% and terminal 1% of bits, n is 20, N is 20 and M is from 1 to 20.

Preferably, the M percent of random selections in step (d) that have the highest scores, or the M' percent of formulations in step (f) that have the lowest IC₅₀ and/or the EC₅₀ against said target, as determined in step (f1), is that percent of each population that exhibits synergistic cosmetic effects. Subsequently, the formulation(s) made in step (e) or the formulation(s) selected in step (f) may be made using different concentrations of each individual ingredient, before determining the IC₅₀ and/or the EC₅₀ of said new formulations against the target referred to in steps (c2b) or (c13b), according to the procedure in step (a). The formula showing the best activity *in vitro* or *in vivo* would then be reported.

Thus, the invention provides an automated method for a rational design of formulas that involves a reduction in cost and time invested in the product development. Moreover, since a whole activity profile for all the biological targets within the system is performed for every single ingredient, instead of an application-specific confirmatory assay as usual in another approaches, an unexpected result may be achieved.

### Examples

### A. Materials and methods:

### 1. Cell cultures.

The cell lines HaCaT (keratinocytes), HaCaT-KBF-Luc, NIH-3T3-KBF-Luc, 3T3-L1 (adipocytes) 293T, 293T-TRPV-1, 293T-CB1, 293T-CB2, are included in the SimDerma platform and maintained in supplemented DMEM medium containing 10% FBS and 1% antibiotics penicillin/streptomycin (DMEM complete medium) at 37 °C in a humidified atmosphere of 5% CO₂.

### 2. Cytotoxicity assays.

The cytotoxic effect of the test substances is investigated by the MTT (3-[4,5-dimethylthiazol-2-yl]-2,5-diphenyltetrazolium bromide) assay. Briefly, NHDFs and HaCaT cells are seeded at a density of 10⁴ cells/well in 96-well plates (100 µL cell suspension per well). After 24 hours the cells are treated with increasing concentrations of the test substances for 24 hours. Subsequently, 50 µL of MTT (5 mg/mL) from a mixture solution of MTT: DMEM (1:2) is added to each well and incubated for 4 hours at 37 °C in darkness. Then the reaction is stopped, supernatant is removed and 100 µL of DMSO is added to each well and incubated for 10 minutes with gentle shaking. Finally, the absorbance is measured at 550 nm using a TECAN GENios Pro plate reader (Tecan Group Ltd, Switzerland). Results are expressed as fold induction calculated against a control sample (vehicle solution, 100%).

B. Examples:

*Example 1: Production of novel and bioactive formulations for treating atopic dermatitis.*

Atopic dermatitis (AD) is an itchy, chronic, and/or chronically relapsing, inflammatory dermatosis, its rash is characterized by itchy papules, occasionally vesicles which become excoriated and lichenified, and typically have a flexural distribution. Although etiopathogenic factors are not completely understood, immunologic, genetic and environmental factors are interrelated to produce a skin barrier disturbance as well as an immunologic dysregulation.

The goal standards for the management of atopic skin treatment include the maintenance of epidermal barrier integrity and the treatment of inflammation and itching. The disruption of epidermal barrier can be treated with PPARα agonists and prevented with moisturizing and antioxidants agents. The inflammation associated with AD is mediated by activation of the dermal immune system and immune-suppressors such as corticoids and tacrolimus are approved therapies for the treatment of AD. Skin itching is a very complex pathological condition and so far no effective treatments have been developed for its use in atopic skin. It has been shown recently that the ionotropic sensory receptors TRPA1 and TRPV-1 are involved in the physiopathology of pruritus. Thus, TRPA1 or TRPV-1 antagonists may be developed as anti-itching agents.

Using the SimDerma database containing more than 100 studied ingredients and the INCOS software, each referred to in the method of the present invention, a novel formulation with synergistic activities on atopic skin targets was identified. The cosmetic use "atopic skin care" was parameterized according to the following requirements related to the experimental targets:
(i) Skin barrier improvement: PPARα induction
(ii) Anti-inflammatory effect: NF-κB inhibition
(iii) Antioxidant effect: Nrf2 induction and inhibition of reactive oxygen species (ROS) induction
(iv) Soothing effect: blocking of TRPV1 channels.

A query was performed with the following settings:
(a) Primary filters: no cost restrictions, no supplier restrictions, only clinically proven ingredients
(b) No preferred organoleptic properties
(c) Main applications: atopic skin care
(d) Preferred additional effects: moisturizing effect
(e) Maximum number of ingredients in the mixture: 5.

The resulting formulation (F01) obtained according to the method of the present invention consist of a mixture of four ingredients each comprising the biologically-active compounds shown in Table 1. Media comprising said formulation were prepared at two different concentrations thereof: 1/10 and 1/100, wherein 1/10 refers to one part (by volume) of solute in nine parts of solvent and 1/100 refers to one part (by volume) of solute in ninety-nine parts of solvent, said solute being the formulation of ingredients and said solvent the culture medium in which the reaction was performed.

**Table 1. Composition of formulation F01.**

| **Compound or composition** | **Active compound(s)** | **F01 (1/10)** | **F01 (1/100)** |
|---|---|---|---|
| A | xymenynic acid | 0.02% (vol./vol.) | 0.002% (vol./vol.) |
| B | alkyl amides | 0.00052% (vol./vol.) | 0.000052% (vol./vol.) |
| C | fatty acids | 0.01 % (vol./vol.) | 0.001% (vol./vol.) |
| | fatty alcohols | | |
| | total sterols | | |
| | beta-sitosterol | | |
| D | pomiferine | 0.1 % (wt./vol.) | 0.01 % (wt./vol.) |
| | osajine | | |
| | other isoflavones | | |

Scores for each of the ingredients of said formulation F01 were established as follows: if no biological activity is found at any concentration against a given target, a value 0 is assigned; if activity is found only at the highest concentration (50 µM) against said given target, a value 1 is assigned; if activity is found at the intermediate concentration (25 µM) against said given target but not at the lowest concentration (10 µM), a value 2 is assigned; and if activity is found at the lowest concentration (10 µM) against said given target, a value 3 is assigned. For each ingredient a mean score was established by averaging the scores against all targets tested (see Table 2). Other scoring systems may be used, such as wherein a mean score is assigned to each ingredient based on the average of at least one score, wherein each score is a percentage of a measured biological activity against a given target.

**Table 2. Scores of biological activities of ingredients of formulation F01 against targets.**

| **Biological activity** | **Score** | | | |
|---|---|---|---|---|
| | **Compound A** | **Composition B** | **Composition C** | **Composition D** |
| Total antioxidant capacity | 2 | 1 | 1 | |
| PPARγ activation | | 3 | | |
| PPARα activation | | 3 | 1 | 1 |
| Glucose uptake | | 3 | 1 | 1 |
| Nrf2 induction | | 2 | | 3 |
| Cannabinoid receptor CB2 activation | | 1 | 1 | |
| NFkB (keratinocyte) (HaCaT) | | 1 | 1 | 3 |
| TRPV1 agonism | | 2 | | 2 |
| NFkB (fibroblast) | | | 1 | 3 |
| Collagen type I α induction | | | 1 | |
| Nrf2 antagonism | | | 1 | |
| STAT3 | | | 1 | 3 |
| TRPV1 antagonism | | | | 1 |
| Melanogenesis | | | | 1 |
| **Mean score** | **2** | **2** | **1** | **2** |
| **Percentage of maximum score** | **66.7** | **66.7** | **33.3** | **66.7** |

The overall score of said formulation F01 was determined from the average of the mean scores of each ingredient as a percentage of the maximum score = 58%. Based on the aforementioned biological activities and the results from the following Examples, said formulation F01 exhibits skin barrier enhancement (PPARα agonism, Example 2), anti-inflammatory activity (NF-κB inhibition, Example 3), anti-oxidant capacity (ROS inhibition, Example 4) and soothing effects (TRPV-1-antagonism, Example 5).

### Example 2. PPARα agonistic activity.

PPARα transactivation assays were performed in NIH-3T3 fibroblasts cells in order to determine the biological activity of formulation F01 on PPARα transcription.

NIH-3T3 cells were transiently co-transfected with the expression vector GAL4-PPARα and the luciferase reporter vector GAL4-Luc using Roti^{©}-Fect (Carl Roth, Karlsruhe, Germany) following manufacturer's instructions. 24 hours after transfection the cells are treated for 12 hours with increasing concentrations of formulation F01. Protein lysates were prepared in a buffer containing 25 mM Tris-phosphate (pH 7.8), 8mM MgCl₂, 1mM DTT, 1% Triton X-100 and 7% glycerol, and luciferase activity was determined using a luciferase assay kit (Promega, Madison, WI, USA), in an Autolumat LB 9510 (Berthold) following the instructions of the luciferase assay kit (Promega, Madison, WI, USA). Specific transactivation is expressed as fold induction over the control (untreated cells), as shown in Figure 5.

### Example 3. NF-κB inhibitory activity of formulation F01.

NIH-3T3-KBF-luc (Figure 6A) and HaCaT-KBF-luc cells (Figure 6B) were stably transfected with the plasmid KBF-Luc plasmid, which contains three copies of NF-κB binding site (from major histocompatibility complex promoter), fused to a minimal simian virus 40 promoter driving the luciferase gene. Cells (15 x 10⁴ cells/mL) were seeded the day before the assay. Then the cells were treated with increasing concentrations of formulation F01 for 15 minutes and then stimulated with TNFα (30 ng/mL). After 6 hours, the cells were washed twice with PBS and lysed in 100 µL lysis buffer containing 25 mM tris-phosphate (pH 7.8), 8 mM MgCl₂, 1 mM DTT, 1% Triton X-100, and 7% glycerol over 15 minutes at 20 - 25 °C in a horizontal shaker. After centrifugation, the supernatant was used to measure luciferase activity using an Autolumat LB 9510 (Berthold) following the instructions of the luciferase assay kit (Promega, Madison, WI, USA). The protein concentration in the cell extracts was measured by the Bradford method (BioRad). The RLU/µg is calculated and the results are expressed as percentage of inhibition of NF-κB activity induced by TNFα (100% activation), as shown in Figures 6A and 6B.

### Example 4. Cellular antioxidant activity.

The intracellular accumulation of ROS was detected by fluorimetry using 2',7'-dihydrofluorescein-diacetate (DCFH-DA, 1 µM). The HaCaT cells (1 x 10⁵ cells/well) were cultured in a 12-well plate in DMEM supplemented with 10% FBS and culture medium was renewed once the cells reached 80% confluence. After pre-incubation with increasing concentrations of formulation F01 or with *N*-acetyl cysteine (NAC, 15 mM) for 15 minutes, the cells were incubated with 10 µM DCFH-DA in the culture medium at 37 °C for 30 minutes and then treated with *tert*-butyl-hydroperoxide (TBHP, 0.4 mM). The acetate groups on DCFH-DA are removed by an intracellular esterase, trapping the probe inside the HaCaT cells. The cells were then washed with warm PBS buffer. The production of ROS was measured by changes in fluorescence due to the intracellular accumulation of DCF caused by the oxidation of DCFH. Intracellular ROS, as indicated by DCF fluorescence, were detected using a TECAN Pro plate reader (excitation, 485 nm; emission, 530 nm) and the results were represented, as shown in Figure 7, as a fold induction of fluorescence (y-axis) taking the control sample (-) as a reference. Data are expressed as mean ± S.D. of at least three independent experiments.

### Example 5. Calcium live-cell imaging as measure of TRPV-1 antagonistic activity.

293T-TRPV-1 cells are stably transfected cells which over-express the human TRPV-1 receptor. 2 x 10⁴ 293T-VR1 cells (or parental 293T cells as control) were cultured in triplicate in 96-well microtiter plates (Costar, Cambridge, MA) in 200 µL of complete medium. Cells in separate wells were incubated with the formulation F01 (1/10 and 1/20 vol./vol.) or with capsazepine (CPZ, as the positive control of TRPV-1 antagonism) for 30 minutes and then stimulated with the TRPV-1 agonist capsaicin (3 µM) for 6 hours in order to allow the entry of extracellular calcium inside the cells. Subsequently, the cellular viability was measured by the MTT method as described above.

Fura2 Ca²⁺ imaging experiments were performed on a BD Pathway 855 High Content Imaging System (Becton Dickinson Biosciences, Heidelberg, Germany). 293T-TRPV1 cells were seeded in 96-well imaging plate (Becton Dickinson Bioscience) the day before the experiment in a density of 5000 cells/well and loaded with 5 µM Fura2-AM (Molecular Probes, Darmstadt, Germany) in buffer containing 135 mM NaCl, 3 mM KCI, 10 mM HEPES, 15 mM D-glucose, 2 mM MgSO₄ and 2 mM CaCl₂ for 30 minutes before the experiment. Calcium mobilization was measured with excitation at 340 and 380 nm for ratiometric analysis and pictures were taken with a delay of 5 seconds. After 5 minutes of recording, ionomycin (1 µg/mL) was added to standardize the results which are shown in Figure 8.

## Claims

1. A method for producing at least one topical dermal formulation for a given cosmetic use, comprising the following steps:
**(a)** determining the IC₅₀ of ingredients which exhibit antagonistic biological activity against at least one target which, when inhibited, effects a cosmetic use, and/or determining the EC₅₀ of ingredients which exhibit agonistic biological activity against at least one target which, when activated, effects a cosmetic use, wherein each of said ingredients may be a compound or a composition, and wherein said target is selected from at least one molecule, protein, protein complex, nucleic acid or nucleic acid complex;
**(b)** compiling an ingredient database comprising data on each of said ingredients, wherein the data for any given ingredient encodes at least one qualitative characteristic of said ingredient and at least one quantitative characteristic of said ingredient, wherein:
- a qualitative characteristic is selected from a qualitative chemical property of said given ingredient, a qualitative physical property of said given ingredient, an organoleptic property of said given ingredient, a supplier of said given ingredient, or a dermatological test performed using said given ingredient; and
- a quantitative characteristic is selected from an IC₅₀ or EC₅₀ value of said given ingredient determined in step (a), a skin permeability value of said given ingredient, a cost per unit weight of said given ingredient, a quantitative chemical property of said given ingredient, or a quantitative physical property of said given ingredient;
**(c)** either:
**(c1)** identifying those ingredients which have data encoding at least one first given qualitative characteristic of said ingredients in said ingredient database, wherein said first given qualitative characteristic is selected from a presence or absence of a given qualitative chemical property, a presence or absence of a given qualitative physical property, a presence or absence of a given organoleptic property, the provenance or not from a given supplier, or a presence or absence of passing a given dermatological test performed;
**(c2)**
**(c2a)** making a random selection of two or more of said ingredients identified in step (c1) for combination into a formulation for said given cosmetic use; and
**(c2b)** calculating the total cost per unit weight of said formulation when all ingredients in said random selection of ingredients made in step (c2a) exhibit:
- antagonistic biological activity (AN) against a given target or targets, wherein each given target is that which, when inhibited in an experimental skin model, effects said given cosmetic use, or
- agonistic biological activity (AG) against a given target or targets, wherein each given target is that which, when activated in an experimental skin model, effects said given cosmetic use,
by multiplying the cost per unit weight of each ingredient in the random selection made in step (c2a) by the concentration (C) at which it is to be combined into said formulation, wherein the concentration (C) for any given ingredient in said random selection is:
- the IC₅₀ value of said ingredient determined in step (a) against said given target, or the maximum of the IC₅₀ values of said ingredient determined in step (a) against said given targets, when said ingredient exhibits said antagonistic biological activity (AN), or
- the EC₅₀ value of said ingredient determined in step (a) against said given target, or the maximum of the EC₅₀ values of said ingredient determined in step (a) against said given targets, when said ingredient exhibits said agonistic biological activity (AG); and
**(c2c)** randomly varying said random selection if the formulation to be made from combining said random selection of ingredients at the concentrations (C) referred to in step (c2b):
- comprises an ingredient that does not have at least one second given qualitative characteristic, wherein said second given qualitative characteristic is different from the at least one first given qualitative characteristic in step (c1), and wherein said second given qualitative characteristic is selected from a presence or absence of a given qualitative chemical property, a presence or absence of a given qualitative physical property, a presence or absence of a given organoleptic property, a presence or absence of a given supplier, or a presence or absence of passing a given dermatological test performed;
- comprises an ingredient which does not exhibit the antagonistic biological activity (AN) or the agonistic biological activity (AG) referred to in step (c2b); and/or
- does not have a total cost per unit weight calculated according to step (c2b) which is less than a given total cost per unit weight,
by:
**(c2ci)** randomly deselecting one of said ingredients from said random selection, provided that said random selection comprises more than two ingredients, thus decreasing the number of ingredients for combination into said formulation;
**(c2cii)** randomly selecting a further ingredient identified in step (c1) for combination with said random selection, thus increasing the number of ingredients for combination into said formulation;
**(c2ciii)** randomly deselecting one of said ingredients from said random selection, and randomly selecting another ingredient identified in step (c1), for combination with the remaining ingredient or ingredients of said random selection, thus maintaining constant the number of ingredients for combination into said formulation; or
**(c2civ)** randomly deselecting a number, x, of said ingredients from said random selection, and randomly selecting a further x ingredients from any of the random selections generated in step (c5), for combination with the remaining ingredient or ingredients of said random selection, provided that said random selection comprises more than (x + 1) ingredients, thus maintaining constant the number of ingredients for combination into said formulation; and
**(c2d)** repeating steps (c2b) and (c2c) n times, wherein n is a whole number less than 100, until:
- all ingredients comprised in said random selection of ingredients have said at least one second given qualitative characteristic,
- all ingredients comprised in said random selection of ingredients exhibit the antagonistic biological activity (AN) or the agonistic biological activity (AG) referred to in step (c2b), and
- said formulation to be made from combining said random selection of ingredients has a total cost per unit weight calculated according to step (c2b) which is less than said given total cost per unit weight;
**(c3)** determining the score of said random selection of two or more ingredients for combination into said formulation, wherein said score is determined by:
- averaging the mean score of each of the ingredients comprised in said random selection, wherein said mean score of any given ingredient is determined by averaging the percentage of a value of at least one quantitative characteristic of said given ingredient at the concentration (C) referred to in step (c2b), relative to a corresponding predetermined value of said at least one quantitative characteristic, and/or determining the percentage of a value of at least one quantitative characteristic of said formulation relative to a corresponding predetermined value of said at least one quantitative characteristic; and/or
- averaging the mean score of each of the ingredients comprised in said random selection, wherein said mean score of any given ingredient is determined by averaging the percentage of an inverse value of at least one quantitative characteristic of said given ingredient at the concentrations (C) referred to in step (c2b), relative to an inverse of a corresponding predetermined value of said at least one quantitative characteristic, and/or determining the percentage of an inverse value of at least one quantitative characteristic of said formulation relative to an inverse of a corresponding predetermined value of said at least one quantitative characteristic;
wherein said quantitative characteristic of each ingredient is selected from an IC₅₀ or EC₅₀ value of said ingredient determined in step (a), a skin permeability value of said ingredient, a cost per unit weight of said ingredient, a quantitative chemical property of said ingredient, or a quantitative physical property of said ingredient, and
wherein said quantitative characteristic of said formulation is selected from a total cost per unit weight of said formulation, a total number of ingredients comprised in said formulation, an IC₅₀ or EC₅₀ value of said formulation against at least one target, a quantitative chemical property of said formulation, or a quantitative physical property of said formulation, and
wherein when an ingredient in said random selection of two or more ingredients does not exhibit a given quantitative characteristic, the percentage assigned thereto is zero;
**(c4)** compiling a formulation database comprising data on said random selection made in step (c2), wherein said data encodes:
- the type and number of ingredients comprised in said random selection and their respective quantities;
- the score of said random selection determined in step (c3);
- the total cost per unit weight of said formulation to be made from combining said random selection of ingredients; and
- the qualitative characteristics and quantitative characteristics of the ingredients comprised therein, wherein said qualitative characteristics and said quantitative characteristics are those compiled in step (b);
**(c5)**
**(c5a)** randomly varying said random selection for which data is compiled in said formulation database to generate a new random selection for combination into a new formulation for said given cosmetic use by:
**(c5ai)** randomly deselecting one of said ingredients from said random selection, provided that said given random selection comprises more than two ingredients, thus decreasing the number of ingredients for combination into said formulation;
**(c5aii)** randomly selecting a further ingredient identified in step (c1) for combination with said random selection, thus increasing the number of ingredients for combination into said formulation;
**(c5aiii)** randomly deselecting one of said ingredients from said random selection, and randomly selecting a further ingredient identified in step (c1), for combination with the remaining ingredient or ingredients of said random selection, thus maintaining constant the number of ingredients for combination into said formulation; or
**(c5aiv)** randomly deselecting a number, x, of said ingredients from said random selection, and randomly selecting a further x ingredients from any of the random selections generated in step (c8), for combination with the remaining ingredient or ingredients of said random selection, provided that said random selection comprises more than (x + 1) ingredients, thus maintaining constant the number of ingredients for combination into said formulation;
**(c5b)** calculating the total cost per unit weight of said new formulation according to step (c2b), provided that all ingredients comprised in said new random selection of two or more ingredients obtained in step (c5a) exhibit the antagonistic biological activity (AN) or the agonistic biological activity (AG) referred to in step (c2b); and
**(c5c)** determining the score of said new random selection of two or more ingredients obtained in step (c5a) for combination into said new formulation according to step (c3);
**(c6)** replacing the data on said random selection in said formulation database with the corresponding data on said new random selection if:
- the score of said new random selection determined in step (c5c) is greater than the score of said random selection in said formulation database;
- said new random selection comprises an ingredient that has the at least one second given qualitative characteristic referred to in step (c2c);
- said new random selection comprises an ingredient which exhibits the antagonistic biological activity (AN) or the agonistic biological activity (AG) referred to in step (c2b); and/or
- said new random selection has a total cost per unit weight calculated according to step (c5b) which is less than said given total cost per unit weight,
**(c7)** repeating steps (c5) and (c6) N times, wherein N is a whole number less than 100;
**(c8)** optionally repeating steps (c2) to (c7) S times to afford S random selections of two or more of said ingredients for combination into S respective formulations, wherein S is a whole number less than 1000;
or
**(c9)** identifying those ingredients which have data encoding at least one given qualitative characteristic of said ingredients in said ingredient database wherein, for a given ingredient, said given qualitative characteristic is selected from a presence or absence of a given qualitative chemical property of said given ingredient, a presence or absence of a given qualitative physical property of said given ingredient, a presence or absence of a given organoleptic property of said given ingredient, or the provenance or not from a given supplier of said given ingredient;
**(c10)** generating 2^{T} binary sequences, wherein each binary sequence reads from left to right and comprises a tuple, T, of bits, wherein T is calculated by rounding up to the nearest whole number the result of log₂(NI), wherein NI is the number of ingredients identified in step (c9), wherein each bit is additionally assigned a value between 0 and 0.1 corresponding to the probability that said bit changes;
**(c11)** assigning each of the ingredients identified in step (c9) one of the binary sequences generated in step (c10);
**(c12)** generating a population of S composite binary sequences, wherein each composite binary sequence reads from left to right and comprises P binary sequences reading from left to right and joined in linear sequence, and wherein
- each of the P binary sequences is randomly selected from the 2^{T} binary sequences generated in step (c10);
- S is a whole number less than 1000; and
- P is a given whole number between 2 and 20 which refers to the maximum possible number of ingredients in said formulation for said given cosmetic use;
**(c13)** for each composite binary sequence:
**(c13a)** identifying which of the ingredients identified in step (c9) are encoded by the P binary sequences comprised therein for combination into a formulation encoded by said composite binary sequence;
**(c13b)** calculating the total cost per unit weight of the formulation encoded by said composite binary sequence, provided that at least two ingredients were identified in step (c13a) and all ingredients identified in step (c13a) exhibit:
- antagonistic biological activity (AN) against a given target or targets, wherein each given target is that which, when inhibited in an experimental skin model, effects said given cosmetic use, or
- agonistic biological activity (AG) against a given target or targets, wherein each given target is that which, when activated in an experimental skin model, effects said given cosmetic use,
by multiplying the cost per unit weight of each ingredient identified in step (c13a) by the concentration (C) at which it is to be combined into the formulation encoded by said composite binary sequence, wherein the concentration (C) for any given ingredient identified in step (c13a) is:
- the IC₅₀ value of said given ingredient as determined in step (a) against said given target, or the maximum of the IC₅₀ values of said ingredient determined in step (a) against said given targets, when said ingredient exhibits said antagonistic biological activity (AN), or
- the EC₅₀ value of said given ingredient as determined in step (a) against said given target, or the maximum of the EC₅₀ values of said ingredient determined in step (a) against said given targets, when said ingredient exhibits said agonistic biological activity (AG); and
**(c13c)** determining the score of the formulation encoded by said composite binary sequence, wherein said score is determined by:
- averaging the mean score of each of the ingredients identified in step (c13a), wherein said mean score of any given ingredient is determined by averaging the percentage of a value of at least one quantitative characteristic of said given ingredient relative to a corresponding predetermined value of said at least one quantitative characteristic, and/or determining the percentage of a value of at least one quantitative characteristic of the formulation encoded by said composite binary sequence relative to a corresponding predetermined value of said at least one quantitative characteristic; and/or
- averaging the mean score of each of the ingredients identified in step (c13a), wherein said mean score of any given ingredient is determined by averaging the percentage of an inverse value of at least one quantitative characteristic of said given ingredient relative to an inverse of a corresponding predetermined value of said at least one quantitative characteristic, and/or determining the percentage of an inverse value of at least one quantitative characteristic of the formulation encoded by said composite binary sequence relative to an inverse of a corresponding predetermined value of said at least one quantitative characteristic;
wherein said quantitative characteristic of each ingredient identified in step (c13a) is selected from an IC₅₀ or EC₅₀ value of said ingredient determined in step (a), a skin permeability value of said ingredient, a cost per unit weight of said ingredient, a quantitative chemical property of said ingredient or a quantitative physical property of said ingredient, and
wherein said quantitative characteristic of the formulation encoded by said composite binary sequence is selected from a cost per unit weight of said formulation, a total number of ingredients comprised in said formulation, an IC₅₀ or EC₅₀ value of said formulation against at least one target, a quantitative chemical property of said formulation, or a quantitative physical property of said formulation and
wherein when:
- an ingredient identified in step (c13a) does not exhibit a given quantitative characteristic, the percentage assigned thereto is zero; and -less than two ingredients were identified in step (c13a) the score of the formulation encoded by said composite binary sequence is zero;
**(c14)** compiling a composite binary sequences database comprising data on each of the composite binary sequences in said population of S composite binary sequences, wherein, for any given composite binary sequence, said data encodes
- the type and number of ingredients in the formulation encoded by said composite binary sequence and their respective quantities;
- the score of said binary sequence determined in step (c13c);
- the cost per unit weight of the formulation encoded by said composite binary sequence; and
- the qualitative characteristics and quantitative characteristics of the ingredients in the formulation encoded by said composite binary sequence, wherein said qualitative characteristics and said quantitative characteristics are those compiled in step (b);
**(c15)**
**(c15a)** duplicating the sub-population of composite binary sequences which encode a formulation having a score in the upper 50% of the population of S formulations;
**(c15b)** randomly interchanging the terminal F% of bits in each composite binary sequence that is comprised in said duplicate sub-population of composite binary sequences with the terminal F% of bits from any other composite binary sequence in said duplicate sub-population of composite binary sequences, thus generating S/2 new composite binary sequences;
**(c15c)** replacing the data for the sub-population of composite binary sequences in said composite binary sequence database which encode a formulation having a score in the lower 50% of the population of S formulations, with data on S/2 new composite binary sequences which were generated in step (c15b);
**(c15d)** optionally varying the value of one randomly selected bit in each composite binary sequence comprised in
- the sub-population of composite binary sequences which encode a formulation having a score in the upper 50% of the population of S formulations, and
- the S/2 new composite binary sequences generated in step (c15b),
wherein the probability that any given bit changes value is between 0 and 0.1, thus generating a new population of S composite binary sequences;
**(c16)** repeating steps (c13) and (c14) for the new population of S composite binary sequences generated in step (c15);
**(c17)** repeating steps (c15) and (c16) K times, wherein K is a whole number less than 1000, or until the average score of the formulations encoded by said composite binary sequence is not increased between consecutive repetitions of steps (c15) and (c16);
**(d)**
**(d1)** selecting:
- the random selection for combination into a formulation obtained in step (c7); or
- the M percent of the random selections for combination into formulations obtained in step (c8) having the highest scores, wherein M is a whole number from 1 to 50,
as the random selection or random selections of ingredients for combination into said at least one topical dermal formulation for cosmetic use, or
**(d2)** selecting:
- the composite binary sequence obtained in step (c17) encoding the formulation having the highest score; or
- the M percent of the composite binary sequences obtained in step (c17) encoding the formulations having the highest scores, wherein M is a whole number from 1 to 50,
as the composite binary sequence or composite binary sequences encoding said at least one topical dermal formulation for cosmetic use; and
**(e)** making said at least one topical dermal formulation for cosmetic use by mixing the ingredients:
- comprised in the random selection or random selections, or
- encoded by the composite binary sequence or composite binary sequences selected in step (d).

2. The method according to claim 1, wherein:
(a) the mean score for any given ingredient for combination into a formulation is determined by averaging the following parameters:
(i) the percentage of:
- an assigned value based on the IC₅₀ value of said given ingredient in said formulation referred to in steps (c2a), (c5a) or (c13a) against at least one given target, relative to a maximum possible assigned value, wherein a value of 0 may be assigned to said given ingredient against a given target when it is not inhibited at any concentration *in vitro,* a value of 1 may be assigned to said given ingredient when inhibition is observed at a concentration falling above the upper quartile of the IC₅₀ values for each ingredient in all of said n formulations against said at least one given target; a value of 2 may be assigned to said given ingredient when inhibition is observed at a concentration falling between the upper and lower quartiles of the IC₅₀ values for each ingredient in all of said n formulations against said at least one given target, and a value of 3 (the maximum possible assigned value) may be assigned when inhibition is observed at a concentration falling below the lower quartile of the IC₅₀ values for each ingredient in all of said n formulations against said at least one given target; or
- the inverse of the IC₅₀ value of said given ingredient in said formulation referred to in steps (c2a), (c5a) or (c13a) against at least one given target, relative to the inverse of the maximum IC₅₀ value against said at least one given target for an ingredient in any of said n formulations;
(ii) the percentage of:
- an assigned value based on the EC₅₀ value of said given ingredient in said formulation referred to in steps (c2a), (c5a) or (c13a) against at least one given target, relative to a maximum possible assigned value, wherein a value of 0 may be assigned to said given ingredient against a given target when it is not activated/induced at any concentration *in vitro,* a value of 1 may be assigned to said given ingredient when activation/induction is observed at a concentration falling above the upper quartile of the EC₅₀ values for each ingredient in all of said n formulations against said at least one given target; a value of 2 may be assigned to said given ingredient when activation/induction is observed at a concentration falling between the upper and lower quartiles of the EC₅₀ values for each ingredient in all of said n formulations against said at least one given target, and a value of 3 (the maximum possible assigned value) may be assigned when activation/induction is observed at a concentration falling below the lower quartile of the EC₅₀ values for an ingredient in any of said n formulations against said at least one given target; or
- the inverse of the mean EC₅₀ value of said given ingredient in said formulation referred to in steps (c2a), (c5a) or (c13a) against at least one given target, relative to the inverse of the maximum EC₅₀ value against said at least one given target for an ingredient in any of said n formulations;
(iii) the percentage of the inverse cost per unit weight of said ingredient, relative to the inverse cost per unit weight of the highest cost per unit weight ingredient in any of said n formulations; and/or
(iv) the percentage of the skin permeability value of said ingredient, relative to the maximum skin permeability value for an ingredient in any of said n formulations; and
(b) the score for any given formulation is determined by calculating:
(v) the percentage of the number of cosmetic uses of said formulation, relative to the greatest number of cosmetic uses of said n formulations;
(vi) the percentage of the number of targeted cosmetic uses of said formulation relative to the maximum number of targeted cosmetic uses;
(vii) the percentage of the number of ingredients identified in step (c1) or step (c9) which are comprised in said formulation, relative to the maximum number of ingredients permissible in said formulation;
(viii) the percentage of the number of targets against which said formulation exhibits an IC₅₀ or EC₅₀ value, relative to the total number of said targets; and/or
(iv) the percentage of the inverse cost per unit weight of said formulation, relative to the inverse cost per unit weight of the lowest cost per unit weight formulation.

3. The method according to claim 2, wherein each of said parameters is optionally independently weighted by a value between 0 and 1.

4. The method according to claim 2, wherein each parameter is weighted equally by a value between 0.1 and 1.

5. The method according to any of the preceding claims, which comprises a further step (f), wherein step (f) comprises:
(f1) determining the IC₅₀ and/or the EC₅₀ of said formulation or formulations made in step (e) against said target or targets referred to in steps (c2b) or (c13b), according to the procedure in step (a); and
(f2) selecting the formulation or M' percent of formulations made in step (e) having the lowest IC₅₀ and/or the EC₅₀ against said target, as determined in step (f1), wherein M' is a whole number from 1 to 50, as said at least one topical dermal formulation for cosmetic use.

6. The method according to any of the preceding claims, wherein the biological activity is selected from NF-κB activity in keratinocytes, NF-κB activity in fibroblasts, STAT3 activity, PPARγ activity, PPARα activity, induction of Nrf2 activity, inhibition of Nrf2 activity, Tcf/LET activation, total antioxidant activity, cellular antioxidant activity, glucose uptake in keratinocytes, CB1 agonistic activity, CB2 agonistic activity, TRPV-1 agonistic activity, TRPV-1 antagonistic activity, tyrosinase activity in melanocytes, melanin content in melanocytes, activation of HIF-1α pathway, inhibition of HIF-1α pathway, induction of collagen gene transcription in fibroblasts (COL1A2-Luc), induction of filaggrin gene transcription in keratinocytes, induction of cyclic AMP, inhibition of cyclic AMP, or DNA repairing activity.

7. The method according to claim 6, wherein the biological activity is selected from NF-κB activity in keratinocytes, NF-κB activity in fibroblasts, PPARγ activity, PPARα activity, induction of Nrf2 activity, inhibition of Nrf2 activity, total antioxidant activity, cellular antioxidant activity, TRPV-1 agonistic activity or TRPV-1 antagonistic activity.

8. The method according to any of the preceding claims, wherein n is 50, N is 50.

9. The method according to any of the preceding claims, wherein S is less than 100.

10. The method according to any of the preceding claims, wherein M is a whole number from 1 to 20, S is less than 100, P is less than or equal to 10, the terminal F% of bits is between the terminal 50% and terminal 1% of bits, n is 50 and N is 50.

11. The method according to any of claims 5 to 10, wherein M' is a whole number from 1 to 20, S is less than 100, P is less than or equal to 8, the terminal F% of bits is between the terminal 10% and terminal 1% of bits, n is 20, N is 20 and M is from 1 to 20.

12. The method according to any of the preceding claims, wherein each bit is additionally assigned a value of 0.02 corresponding to the probability that said bit changes.

13. The method according to any of the preceding claims, wherein the experimental skin model is an *in vitro* skin model comprising at least one cell type selected from keratinocytes, melanocytes, Langerhans cells, dermal fibroblasts, dermal dendritic cells, skin resident T-cells or endothelial cells.
